# EUROPEAN PATENT APPLICATION

(11) **EP 4 696 331 A1**
(43) Date of publication of application: **18.02.2026**
(21) Application number: 24788224.4
(22) Date of filing: 12.04.2024
(51) Int. Cl.: A61K 47/68, C07K 16/28, C07D 491/22, A61P 35/00

(54) **ANTI-CDH6 ANTIBODY-DRUG CONJUGATE AND USE THEREOF**

(30) Priority: 14.04.2023 CN 202310420429
(71) Applicant: Hainan Simcere Zaiming Pharmaceutical Co., Ltd., Hainan 570311 (CN)
(72) Inventor: CHAI, Xiaojuan, Shanghai 201318 (CN); FU, Yayuan, Shanghai 201318 (CN); WEI, Menghao, Shanghai 201318 (CN); LI, Zhen, Shanghai 201318 (CN); TANG, Renhong, Shanghai 201318 (CN)
(74) Representative: D Young & Co LLP
(86) International application number: PCT/CN2024/087545
(87) International publication number: WO 2024/213128

(57) **Abstract**

The present invention relates to a class of antibody-drug conjugates with a novel structure. Specifically, disclosed in the present invention is an antibody-drug conjugate targeting CDH6, which has good plasma stability in plasma of mammals (such as humans or monkeys), has a relatively strong anti-tumor effect, and has wide clinical application prospects in the treatment of diseases such as tumors.

## Description

The present disclosure claims priority to the Chinese Patent Application No. 202310420429.5 entitled "ANTI-CDH6 ANTIBODY-DRUG CONJUGATE AND USE THEREOF" and filed with the China National Intellectual Property Administration on April 14, 2023, which is incorporated herein by reference in its entirety.

### TECHNICAL FIELD

The present disclosure pertains to the field of biomedicine, and relates to an antibody-drug conjugate with a novel structure, a preparation method therefor, a pharmaceutical composition comprising the conjugate, and use thereof as an anti-tumor drug.

### BACKGROUND

Antibody-drug conjugate (ADC), as a novel targeted drug, is formed by linking a monoclonal antibody specifically binding to a tumor cell surface antigen to a biologically active toxin molecule, thereby combining the tumor-targeting capability of the antibody with the efficient killing effect of the toxin molecule, while overcoming the former's disadvantage of poor therapeutic effect and the latter's disadvantage of serious toxic and side effects, poor druggability, etc. Compared to traditional chemotherapeutic agents, ADC drugs can accurately target tumor cells and reduce the impact on normal cells so as to achieve safer and more effective anti-tumor effects.

An ADC generally comprises three parts: an antibody, a linker, and a toxin. Camptothecin derivatives are a class of toxins applied to ADC development, which achieve anti-tumor effects by inhibiting topoisomerase I. Daiichi Sankyo employed the camptothecin derivative Dxd as a toxin to develop Enhertu (Trastuzumab deruxtecan, DS-8201), an ADC drug targeting HER2, which was approved for marketing by the U.S. FDA in 2019. Clinical studies have shown that Enhertu exhibits good therapeutic efficacy against HER2-positive breast cancer, gastric cancer, non-small cell lung cancer, etc.

CDH6 is a type II classical cadherin, also known as K-cadherin. CDH6 is a single-pass transmembrane protein composed of 790 amino acids, with its extracellular region divided into 5 subregions (EC1-EC5). Research has demonstrated that CDH6 is predominantly highly expressed in tumor tissues such as renal cancer, ovarian cancer, and thyroid cancer, but is rarely expressed in normal tissues (Cancer Discov; 2017, 7(9): 1030-45). Like other members of the cadherin superfamily, the CDH6 protein is localized to the basolateral membrane of epithelial cells, mediates calcium-dependent cell-cell adhesion, and exhibits rapid internalization properties. Therefore, CDH6 can be used as a potential target for ADC development for the treatment of cancers such as ovarian cancer and renal cancer.

Despite some advancements in the treatment of ovarian cancer and renal cancer in recent years, there are still substantial unmet clinical needs. Currently, only a limited number of CDH6-targeting ADC drugs are undergoing research and development, and no relevant drugs have been approved for marketing. Therefore, the development of ADC drugs directed against this target has broad clinical application and therapeutic prospects.

### SUMMARY

The present disclosure provides an antibody-drug conjugate having a structural general formula of Pc-(L-D)ₙ or a pharmaceutically acceptable salt thereof,
wherein
D is a cytotoxic drug;
L is a linker unit;
Pc is an antibody or an antigen-binding fragment thereof that specifically binds to CDH6;
the antibody or the antigen-binding fragment comprises a heavy chain variable region (VH) or/and a light chain variable region (VL), wherein the heavy chain variable region comprises HCDR1, HCDR2, and HCDR3, or/and the light chain variable region comprises LCDR1, LCDR2, and LCDR3, wherein the HCDR1-3 or/and the LCDR1-3 are selected from the following combinations:
   (1) the HCDR1-3 are SEQ ID NOs: 11-13, or/and the LCDR1-3 are SEQ ID NOs: 14-16;
   (2) the HCDR1-3 are SEQ ID NOs: 17-19, or/and the LCDR1-3 are SEQ ID NOs: 20-22;
   (3) the HCDR1-3 are SEQ ID NOs: 23-25, or/and the LCDR1-3 are SEQ ID NOs: 26-28; or
the HCDR1-3 or/and the LCDR1-3 have a sequence having at least 80% identity or having at most 3 insertion, deletion, or substitution mutations compared to each CDR in the HCDR1-3 and the LCDR1-3 in any one of groups (1)-(3); preferably, the at least 80% identity is 85%, 90%, 91%, 92%, 93%, 94%, 95%, 96%, 97%, 98%, 99%, or 100% identity;
moreover, n is a real number of 1-16.

In some embodiments, the antibody or the antigen-binding fragment comprises a heavy chain variable region (VH) or/and a light chain variable region (VL), wherein the heavy chain variable region or/and the light chain variable region is selected from the following:
(1) the heavy chain variable region is the sequence set forth in SEQ ID NO. 1, or/and the light chain variable region is the sequence set forth in SEQ ID NO. 2;
(2) the heavy chain variable region is the sequence set forth in SEQ ID NO. 3, or/and the light chain variable region is the sequence set forth in SEQ ID NO. 4;
(3) the heavy chain variable region is the sequence set forth in SEQ ID NO. 5, or/and the light chain variable region is the sequence set forth in SEQ ID NO. 6;
   or
the heavy chain variable region or/and the light chain variable region has a sequence having at least 80% identity or having at most 3 insertion, deletion, or substitution mutations compared to the heavy chain variable region or/and the light chain variable region in any one of groups (1)-(3) described above; preferably, the at least 80% identity is 85%, 90%, 91%, 92%, 93%, 94%, 95%, 96%, 97%, 98%, 99%, or 100% identity.

In some embodiments, the antibody or the antigen-binding fragment comprises a heavy chain constant region sequence and/or a light chain constant region sequence; optionally, the heavy chain constant region and/or the light chain constant region is selected from an intact constant region sequence or a fragment thereof, and the constant region fragment includes CH1, a hinge region, CH2, CH3, or Fc; optionally, the heavy chain constant region is selected from human and murine IgG1, IgG2, IgG3, and IgG4 constant regions, and the light chain constant region is selected from human and murine kappa constant regions and lambda constant regions; optionally, the antibody or the antigen-binding fragment comprises an intact heavy chain and light chain, wherein the heavy chain consists of the VH and the heavy chain constant region, the heavy chain constant region having the sequence set forth in SEQ ID NO: 9, and the light chain consists of the VL and the light chain constant region, the light chain constant region having the sequence set forth in SEQ ID NO: 10.

In some embodiments, the antibody or the antigen-binding fragment is:
(1) a chimeric antibody or a fragment thereof;
(2) a humanized antibody or a fragment thereof; and/or
(3) a fully human antibody or a fragment thereof;
preferably, the antibody or the antigen-binding fragment is selected from a monoclonal antibody, a polyclonal antibody, a natural antibody, an engineered antibody, a monospecific antibody, a multispecific antibody (e.g., a bispecific antibody), a monovalent antibody, a multivalent antibody, a full-length antibody, an antibody fragment, a naked antibody, a conjugated antibody, a humanized antibody, a fully human antibody, a Fab, a Fab', an F(ab')2, an Fd, an Fv, an scFv, a diabody, and a single domain antibody.

In some embodiments, the antigen-binding fragment is selected from one or more of an F(ab)2, a Fab', a Fab, an Fv, an scFv, a bispecific antibody, a nanobody, and a minimal recognition unit of an antibody.

In some embodiments, in the antibody-drug conjugate having the general formula of Pc-(L-D)n or the pharmaceutically acceptable salt thereof described above, the cytotoxic drug D is selected from a chemotherapeutic agent and an antibiotic.

In some embodiments, the cytotoxic drug D is selected from a DNA topoisomerase inhibitor.

In some embodiments, the cytotoxic drug D is selected from a compound represented by formula (D-I): 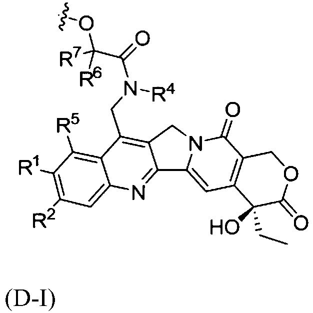 wherein
R¹ and R², together with the atoms to which they are attached, form a 5- to 6-membered heterocyclic ring, wherein the 5- to 6-membered heterocyclic ring comprises 1 or 2 oxygen atoms as ring atoms, and the 5- to 6-membered heterocyclic ring is optionally substituted with one or more D atoms;
R⁴ is selected from H and C₁-C₃ alkyl;
R⁵ is selected from H, halogen, CN, =O, OH, NH₂, and C₁-C₃ alkyl;
R⁶ is selected from H and C₁-C₃ alkyl;
R⁷ is selected from H, C₁-C₃ alkyl, and C₃-C₆ cycloalkyl, wherein the C₁-C₃ alkyl or the C₃-C₆ cycloalkyl is optionally substituted with D, halogen, CN, =O, OH, NH₂, or C₁-C₃ alkyl.

In some embodiments, R¹ and R², together with the atoms to which they are attached, form

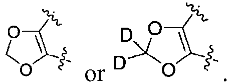

In some embodiments, R⁴ is selected from H.

In some embodiments, R⁵ is selected from H, halogen, CN, OH, NH₂, and C₁-C₃ alkyl.

In some embodiments, R⁵ is selected from H.

In some embodiments, R⁶ is selected from H.

In some embodiments, R⁷ is selected from cyclopropyl.

In some embodiments, the compound represented by formula (D-I) is selected from one of the following compounds: 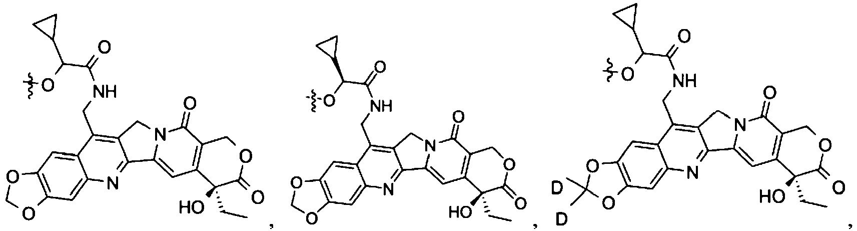 and

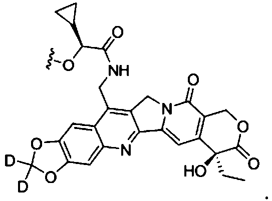

In some embodiments, in the antibody-drug conjugate having the general formula of Pc-(L-D)n or the pharmaceutically acceptable salt thereof described above, the linker unit L is selected from 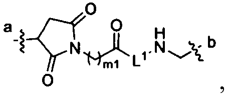 the end a of which is covalently linked to the antibody unit Pc, and the end b of which is covalently linked to the cytotoxic drug D, wherein m1 is an integer selected from 2-8, and L¹ is selected from a peptide residue consisting of 1 to 8 amino acids, wherein the peptide residue is further optionally substituted with one or more substituents selected from halogen, CN, =O, C₁-C₆ alkyl, OH, O(C₁-C₆ alkyl), NH₂, NH(C₁-C₆ alkyl), N(C₁-C₆ alkyl)₂, C₃-C₆ cycloalkyl, and 4- to 7-membered heterocyclyl.

In some embodiments, L¹ is a Gly-Gly-Phe-Gly tetrapeptide residue.

In some embodiments, m1 is 5.

In some embodiments, the linker unit L is 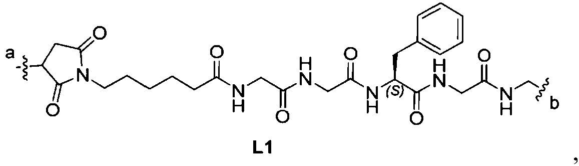 the end a of which is covalently linked to the antibody unit Pc, and the end b of which is covalently linked to the drug unit D.

In some embodiments, in the antibody-drug conjugate having the general formula of Pc-(L-D)n or the pharmaceutically acceptable salt thereof described above, n is a real number selected from 1-16, such as a real number selected from 2-12, such as a real number selected from 4-10, such as a real number selected from 5-9, such as a real number selected from 6-8.

In some embodiments, n is a real number selected from 5-9; for example, n is 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9, or 9.0.

In some embodiments, the antibody-drug conjugate having the general formula of Pc-(L-D)n or the pharmaceutically acceptable salt thereof of the present disclosure is selected from the following compounds or pharmaceutically acceptable salts thereof: 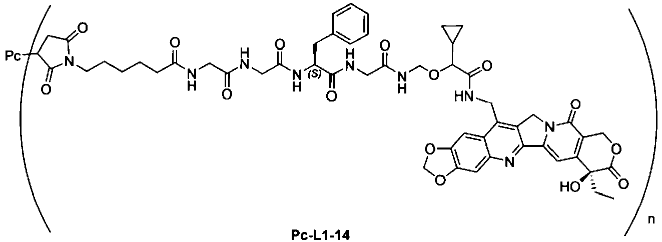 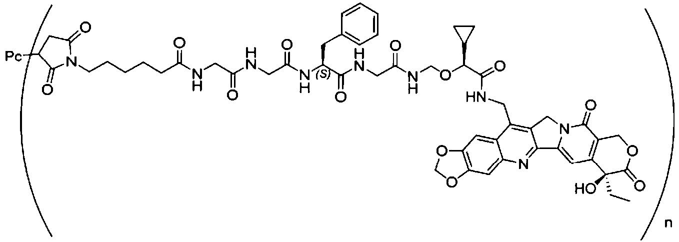 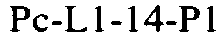 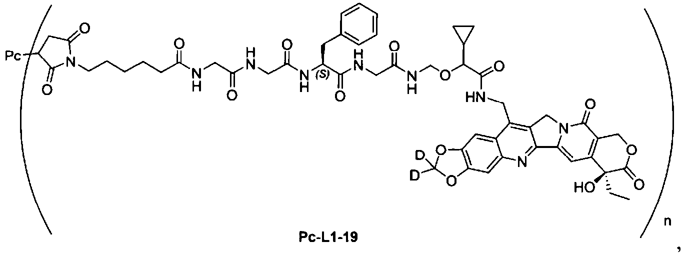 and 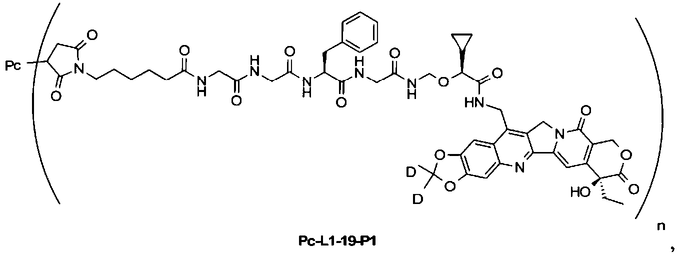 wherein Pc and n are as defined in any one of the preceding embodiments.

In another aspect, the present disclosure provides an isolated nucleic acid molecule encoding the antibody or the antigen-binding fragment thereof described above.

In some embodiments, the present disclosure provides an expression vector comprising the nucleic acid molecule described above.

In some embodiments, the present disclosure provides an isolated host cell of the nucleic acid molecule described above or the expression vector described above; preferably, the host cell is a eukaryotic cell or a prokaryotic cell; more preferably, the host cell is derived from a mammalian cell, a yeast cell, an insect cell, *Escherichia coli,* and/or *Bacillus subtilis;* more preferably, the host cell is selected from an Expi293 cell and a CHO cell.

In another aspect, the present disclosure provides a pharmaceutical composition, comprising the antibody-drug conjugate having the general formula of Pc-(L-D)n or the pharmaceutically acceptable salt thereof described above in the present disclosure, and a pharmaceutically acceptable excipient.

In another aspect, the present disclosure provides a method for treating a tumor in a mammal, comprising administering to a mammal, preferably a human, in need of the treatment a therapeutically effective amount of the antibody-drug conjugate having the general formula of Pc-(L-D)n or the pharmaceutically acceptable salt thereof or the pharmaceutical composition thereof described above.

In some embodiments, the tumor is a CDH6-expressing tumor.

In some embodiments, the tumor is selected from the group consisting of: ovarian cancer, renal cancer, liver cancer, soft tissue cancer, central nervous system cancer, thyroid cancer, and cholangiocarcinoma.

In another aspect, the present disclosure provides use of the antibody-drug conjugate having the general formula of Pc-(L-D)n or the pharmaceutically acceptable salt thereof or the pharmaceutical composition thereof described above in the manufacture of a medicament for treating a tumor.

In some embodiments, the tumor is a CDH6-expressing tumor.

In some embodiments, the tumor is selected from the group consisting of: ovarian cancer, renal cancer, liver cancer, soft tissue cancer, central nervous system cancer, thyroid cancer, and cholangiocarcinoma.

In another aspect, the present disclosure provides use of the antibody-drug conjugate having the general formula of Pc-(L-D)n or the pharmaceutically acceptable salt thereof or the pharmaceutical composition thereof described above in treating a tumor.

In some embodiments, the tumor is a CDH6-expressing tumor.

In some embodiments, the tumor is selected from the group consisting of: ovarian cancer, renal cancer, liver cancer, soft tissue cancer, central nervous system cancer, thyroid cancer, and cholangiocarcinoma.

In another aspect, the present disclosure provides the antibody-drug conjugate having the general formula of Pc-(L-D)n or the pharmaceutically acceptable salt thereof or the pharmaceutical composition thereof described above for use in treating a tumor.

Beneficial effects: The antibody-drug conjugate of the present disclosure can specifically recognize the CDH6 target, demonstrates good plasma stability in mammalian (e.g., human or monkey) plasma, and/or exhibits a relatively strong anti-tumor effect, thereby having broad clinical application prospects in the treatment of diseases such as tumors.

### Terminology and Definitions

Unless otherwise stated, the terms used in the present disclosure have the following meanings, and the definitions of groups and terms described in the present disclosure, including definitions thereof as examples, exemplary definitions, preferred definitions, definitions documented in tables, definitions of specific compounds in the examples, and the like, may be arbitrarily combined and incorporated with each other. A particular term, unless otherwise specifically defined, should not be considered uncertain or unclear, but construed according to its common meaning in the art. When referring to a trade name herein, it is intended to refer to its corresponding commercial product or its active ingredient.

The term "antibody-drug conjugate" (ADC) means that an antibody or an antigen-binding fragment thereof is linked to a biologically active drug via a stable linker unit. The linkage may be achieved by a covalent bond or a non-covalent interaction such as electrostatic force. To form an immunoconjugate, various linkers known in the art may be used.

The term "DAR" or "drug-to-antibody ratio" refers to the average number of small-molecule cytotoxic drugs linked per antibody molecule. In the antibody-drug conjugate of the present disclosure, the DAR is defined by the variable "n", which may be either an integer or a decimal.

The term "antibody" is used in its broadest sense and refers to a polypeptide or a combination of polypeptides that comprises sufficient sequence from an immunoglobulin heavy chain variable region and/or sufficient sequence from an immunoglobulin light chain variable region to be capable of specifically binding to an antigen. The "antibody" herein encompasses various forms and various structures as long as they exhibit the desired antigen-binding activity. The "antibody" herein includes alternative protein scaffolds or artificial scaffolds having grafted complementarity determining regions (CDRs) or CDR derivatives. Such scaffolds include antibody-derived scaffolds comprising mutations introduced to, for example, stabilize the three-dimensional structure of the antibody, and fully synthetic scaffolds comprising, for example, biocompatible polymers. See, e.g., Korndorfer et al., 2003, Proteins: Structure, Function, and Bioinformatics, 53(1): 121-129 (2003); and Roque et al., Biotechnol. Prog. 20:639-654 (2004); the contents of these references are incorporated herein by reference. Such scaffolds may also include non-antibody-derived scaffolds, such as scaffold proteins known in the art to be useful for grafting CDRs, including but not limited to, tenascin, fibronectin, peptide aptamers, and the like.

The "antibody" herein includes a typical "four-chain antibody", which is an immunoglobulin consisting of two heavy chains (HCs) and two light chains (LCs). The heavy chain refers to a polypeptide chain consisting of, from the N-terminus to the C-terminus, a heavy chain variable region (VH), a heavy chain constant region CH1 domain, a hinge region (HR), a heavy chain constant region CH2 domain, and a heavy chain constant region CH3 domain; moreover, when the full-length antibody is of IgE isoform, the heavy chain optionally further comprises a heavy chain constant region CH4 domain. The light chain is a polypeptide chain consisting of, from the N-terminus to the C-terminus, a light chain variable region (VL) and a light chain constant region (CL). The heavy chains are connected to each other and to the light chains through disulfide bonds to form a Y-shaped structure. The heavy chain constant regions of immunoglobulins differ in their amino acid composition and arrangement, and thus in their antigenicity. Accordingly, the "immunoglobulin" herein can be divided into five classes, or isoforms of immunoglobulins, i.e., IgM, IgD, IgG, IgA, and IgE, with their corresponding heavy chains being µ, δ, γ, α, and ε chains, respectively. The Ig of the same class may also be divided into different subclasses according to the differences in the amino acid composition of the hinge regions and the number and location of disulfide bonds in the heavy chains. For example, IgG may be divided into IgG1, IgG2, IgG3, and IgG4, and IgA may be divided into IgA1 and IgA2. Light chains are divided into κ chain or λ chain according to differences in the constant regions. Each of the five classes of Ig may have a κ chain or a λ chain.

The "antibody" herein may be derived from any animal, including, but not limited to, human and non-human animals, wherein the non-human animals may be selected from primates, mammals, rodents, and vertebrates, such as Camelidae species, *Lama glama, Lama guanicoe, Vicugna pacos,* sheep, rabbits, mice, rats, or *Chondrichthyes* (e.g., shark).

The "antibody" herein includes but is not limited to, monoclonal antibodies, polyclonal antibodies, monospecific antibodies, multispecific antibodies (e.g., bispecific antibodies), monovalent antibodies, multivalent antibodies, intact antibodies, fragments of an intact antibody, naked antibodies, conjugated antibodies, chimeric antibodies, humanized antibodies, or fully human antibodies.

The term "monoclonal antibody" refers to an antibody obtained from a population of substantially homogeneous antibodies, that is, the individual antibodies constituting the population are identical and/or bind to the same epitope, except for possible variants (e.g., containing naturally occurring mutations or arising during the production of the formulation, such variants typically being present in minor amounts). In contrast to polyclonal antibody formulations, which generally comprise different antibodies directed against different determinants (epitopes), each monoclonal antibody in a monoclonal antibody formulation is directed against a single determinant on an antigen. The modifier "monoclonal" herein is not to be construed as requiring the antibody or the antigen-binding molecule to be produced by any particular method. For example, monoclonal antibodies can be prepared by a variety of techniques, including (but not limited to) a hybridoma technique, a recombinant DNA method, a phage library display technique, methods that utilize transgenic animals containing all or part of human immunoglobulin loci, and other methods known in the art.

The term "natural antibody" refers to an antibody that is made and paired by the immune system of a multicellular organism. The term "engineered antibody" herein refers to a non-natural antibody obtained by a technique such as genetic engineering and antibody engineering. Illustratively, "engineered antibody" includes humanized antibodies, small-molecule antibodies (e.g., scFv and the like), bispecific antibodies, and the like.

The term "monospecific" means having one or more binding sites, each of which binds to the same epitope of the same antigen.

The term "multispecific antibody" means having at least two antigen-binding sites, each of which binds to a different epitope of the same antigen or a different epitope of a different antigen. Thus, the terms such as "bispecific", "trispecific", and "tetraspecific" refer to the number of different epitopes to which an antibody/antigen-binding molecule can bind.

The term "valent" refers to the presence of a specified number of binding sites in an antibody/antigen-binding molecule. Thus, the terms "monovalent", "divalent", "tetravalent", and "hexavalent" refer to the presence of one binding site, two binding sites, four binding sites, and six binding sites, respectively, in an antibody/antigen-binding molecule.

"Full-length antibody", "complete antibody", and "intact antibody" herein are used interchangeably and refer to an antibody having a structure substantially similar to that of a natural antibody.

"Antigen-binding fragment" and "antibody fragment" herein are used interchangeably and do not have the entire structure of an intact antibody, but comprise only a portion of the intact antibody or a variant of the portion that retains the ability to bind to an antigen. "Antigen-binding fragment" or "antibody fragment" herein includes but is not limited to, a Fab, a Fab', a Fab'-SH, an F(ab')2, an Fv, a VHH, and an scFv.

An intact antibody is digested by papain to produce two identical antigen-binding fragments, called "Fab" fragments, each of which contains a heavy chain variable domain and a light chain variable domain, as well as a light chain constant domain and a first heavy chain constant domain (CH1). Thus, the term "Fab fragment" herein refers to an antibody fragment comprising a light chain fragment comprising the VL domain and the constant domain (CL) of a light chain, and the VH domain and the first constant domain (CH1) of a heavy chain. The Fab' fragment differs from the Fab fragment by the addition of a few residues (including one or more cysteines from an antibody hinge region) at the carboxyl terminus of the heavy chain CH1 domain. Fab'-SH is a Fab' fragment in which the cysteine residue in the constant domain carries a free thiol group. Pepsin treatment produces an F(ab')2 fragment having two antigen-binding sites (two Fab fragments) and a portion of the Fc region. "Fv fragment" is the smallest fragment produced by IgG and IgM, comprising the intact antigen-binding site. The Fv fragment has the same binding properties and similar three-dimensional binding properties as Fab. The VH and VL chains of the Fv fragment are bound together by a non-covalent interaction.

The term "scFv" (single-chain variable fragment) refers to a single polypeptide chain comprising VL and VH domains, wherein the VL and VH are linked through a linker (see, e.g., Bird et al., Science 242:423-426 (1988); Huston et al., Proc. Natl. Acad. Sci. USA 85:5879-5883

(1988); and Pluckthun, The Pharmacology of Monoclonal Antibodies, Vol. 113, Roseburg and Moore Ed., Springer-Verlag, New York, pp 269-315 (1994); the contents of these references are incorporated herein by reference). Such scFv molecules may have a general structure: NH2-VL-linker-VH-COOH or NH2-VH-linker-VL-COOH. An appropriate linker in the prior art consists of GGGGS (SEQ ID NO: 30) amino acid sequence repeats or a variant thereof. For example, a linker having the amino acid sequence (GGGGS)4 (SEQ ID NO: 31) may be used, and variants thereof may also be used (Holliger et al. (1993), Proc. Natl. Acad. Sci. USA, 90:6444-6448, the content of which is incorporated herein by reference). Other linkers that can be used in the present disclosure are described in Alfthan et al. (1995), Protein Eng., 8:725-731; Choi et al. (2001), Eur. J. Immunol., 31:94-106; Hu et al. (1996), Cancer Res., 56:3055-3061, Kipriyanov et al. (1999), J. Mol. Biol., 293:41-56; and Roovers et al. (2001), Cancer Immunol (the contents of these references are incorporated herein by reference). In some cases, there may also be disulfide bonds between the VH and VL of the scFv, forming a disulfide-linked Fv (dsFv).

The term "diabody" refers to an antibody having VH and VL domains that are expressed on a single polypeptide chain, but using a linker that is too short to allow the pairing of the two domains on the same chain, thereby forcing the domains to pair with the complementary domains of the other chain and generating two antigen-binding sites (see, e.g., Holliger P. et al., Proc. Natl. Acad. Sci. USA 90:6444-6448 (1993), and Poljak R.J. et al., Structure 2:1121-1123 (1994); the contents of these references are incorporated herein by reference).

The term "chimeric antibody" refers to an antibody in which a portion of the light chain or/and heavy chain is derived from one antibody (which may be derived from a particular species or belong to a particular antibody class or subclass) and another portion of the light chain or/and heavy chain is derived from another antibody (which may be derived from the same or a different species or belong to the same or a different antibody class or subclass), but which nevertheless retains binding activity to a target antigen (U.S.P 4,816,567 to Cabilly et al.; Morrison et al., Proc. Natl. Acad. Sci. USA, 81:6851 6855(1984)). For example, the term "chimeric antibody" may include an antibody (e.g., a human-murine chimeric antibody) in which the heavy and light chain variable regions of the antibody are derived from a first antibody (e.g., a murine antibody) and the heavy and light chain constant regions of the antibody are derived from a second antibody (e.g., a human antibody).

The term "humanized antibody" refers to a genetically engineered non-human antibody that has an amino acid sequence modified to increase the homology to the sequence of a human antibody. Generally, all or part of the CDRs of a humanized antibody are derived from a non-human antibody (donor antibody), and all or part of the non-CDR regions (e.g., variable region FRs and/or constant regions) are derived from a human immunoglobulin (receptor antibody). The humanized antibody generally retains or partially retains the desired properties of the donor antibody, including, but not limited to, antigen specificity, affinity, reactivity, the ability to increase the activity of immune cells, the ability to enhance immune response, and the like.

The term "fully human antibody" refers to an antibody having variable regions in which both the FRs and CDRs are derived from human germline immunoglobulin sequences. Furthermore, if the antibody comprises constant regions, the constant regions are also derived from human germline immunoglobulin sequences. The fully human antibody herein may include amino acid residues that are not encoded by human germline immunoglobulin sequences (e.g., mutations introduced by random or site-specific mutagenesis *in vitro* or by somatic mutations *in vivo*)*.* However, "fully human antibody" herein does not include antibodies in which CDR sequences derived from the germline of another mammalian species (e.g., mouse) have been grafted onto human framework sequences.

The term "naked antibody" herein refers to an antibody that is not linked, fused, or conjugated to another agent or molecule (e.g., a label or a drug), peptide, or polypeptide. In specific embodiments, a naked antibody expressed by a mammalian host cell may be glycosylated by the host cell's glycosylation machinery (e.g., a glycosylase). In certain embodiments, the naked antibody is not glycosylated when expressed by a host cell lacking its own glycosylation machinery (e.g., a glycosylase). In certain embodiments, the naked antibody is an intact antibody, while in other embodiments, the naked antibody is an antigen-binding fragment of an intact antibody, such as a Fab antibody.

The term "variable region" refers to a region of a heavy or light chain of an antibody involved in the binding of the antibody to an antigen. "Heavy chain variable region" is used interchangeably with "VH" and "HCVR", and "light chain variable region" is used interchangeably with "VL" and "LCVR". The heavy and light chain variable domains (VH and VL, respectively) of natural antibodies generally have similar structures, each of which contains four conserved framework regions (FRs) and three hypervariable regions (HVRs). See, e.g., Kindt et al., Kuby Immunology, 6th ed., W. H. Freeman and Co., p. 91 (2007). A single VH or VL domain may be sufficient to provide antigen-binding specificity. The terms "complementarity determining region" and "CDR" herein are used interchangeably and generally refer to a hypervariable region (HVR) of a heavy chain variable region (VH) or a light chain variable region (VL), which is also known as the complementarity determining region as it is precisely complementary to an antigenic epitope in spatial structure, wherein the heavy chain variable region CDR may be abbreviated as HCDR and the light chain variable region CDR may be abbreviated as LCDR. The terms "framework region" and "FR" are used interchangeably and refer to those amino acid residues of an antibody heavy chain variable region or light chain variable region other than CDRs. Generally, a typical antibody variable region consists of 4 FRs and 3 CDRs in the following order: FR1-CDR1-FR2-CDR2-FR3-CDR3-FR4.

The "CDR" herein may be labeled and defined in a manner well known in the art, including but not limited to the Kabat numbering scheme, Chothia numbering scheme, or IMGT numbering scheme; the tool websites used include, but are not limited to, the AbRSA site (http://cao.labshare.cn/AbRSA/cdrs.php), abYsis site (www.abysis.org/abysis/sequence_input/key _annotation/key_annotation.cgi), and IMGT site (http://www.imgt.org/3Dstructure-DB/cgi/DomainGapAlign.cgi#results). The CDR herein includes overlaps and subsets of amino acid residues defined in different ways.

The term "heavy chain constant region" herein refers to the carboxyl-terminal portion of an antibody heavy chain that is not directly involved in the binding of the antibody to an antigen, but exhibits effector functions, such as interaction with an Fc receptor; it has a more conserved amino acid sequence relative to the variable domains of the antibody. The "heavy chain constant region" at least comprises: a CH1 domain, a hinge region, a CH2 domain, a CH3 domain, or a variant or fragment thereof. The "heavy chain constant region" includes a "full-length heavy chain constant region" having a structure substantially similar to that of a natural antibody constant region and a "heavy chain constant region fragment" including only "a portion of the full-length heavy chain constant region". Illustratively, a typical "full-length antibody heavy chain constant region" consists of the CH1 domain-hinge region-CH2 domain-CH3 domain. When the antibody is IgE, it further comprises a CH4 domain; when the antibody is a heavy chain antibody, it does not comprise a CH1 domain. Illustratively, a typical "heavy chain constant region fragment" may be selected from CH1, Fc, and CH3 domains.

The term "light chain constant region" herein refers to the carboxyl-terminal portion of an antibody light chain that is not directly involved in the binding of the antibody to an antigen. The light chain constant region may be selected from a constant κ domain and a constant λ domain.

The term "Fc" herein refers to the carboxyl-terminal portion of an antibody that is formed by the hydrolysis of an intact antibody by papain; typically, it comprises the CH3 and CH2 domains of the antibody. The Fc region includes, for example, an Fc region of a native sequence, a recombinant Fc region, and a variant Fc region. Although the boundaries of the Fc region of an immunoglobulin heavy chain may vary slightly, the Fc region of a human IgG heavy chain is generally defined as extending from an amino acid residue at position Cys226, or from Pro230, to the carboxyl terminus thereof. The C-terminal lysine of the Fc region (residue 447 according to the Kabat numbering scheme) may be removed, for example, during production or purification of the antibody, or by recombinant engineering of the nucleic acid encoding the heavy chain of the antibody. Therefore, the Fc region may include or exclude Lys447.

The term "single domain antibody" herein refers to a single domain antibody consisting only of a heavy chain variable region, which is obtained by cloning of a variable region of a heavy chain antibody naturally lacking a light chain in a camel.

The term "identity" herein can be calculated as follows: to determine the percent "identity" between two amino acid sequences or two nucleic acid sequences, the sequences are aligned for optimal comparison purposes (e.g., gaps can be introduced into one or both of the first and second amino acid sequences or nucleic acid sequences for optimal alignment, or non-homologous sequences can be discarded for comparison). Amino acid residues or nucleotides at corresponding amino acid positions or nucleotide positions are then compared. When a position in the first sequence is occupied by the same amino acid residue or nucleotide as at the corresponding position in the second sequence, the molecules are identical at this position.

The term "nucleic acid" herein includes any compound and/or substance that comprises a polymer of nucleotides. Each nucleotide consists of a base, in particular a purine or pyrimidine base (i.e., cytosine (C), guanine (G), adenine (A), thymine (T), or uracil (U)), a sugar (i.e., deoxyribose or ribose), and a phosphate group. Generally, a nucleic acid molecule is described as a sequence of bases, whereby the bases represent the primary structure (linear structure) of the nucleic acid molecule. The sequence of bases is generally expressed as 5' to 3'. Herein, the term "nucleic acid molecule" encompasses: deoxyribonucleic acid (DNA), including, e.g., complementary DNA (cDNA) and genomic DNA; ribonucleic acid (RNA), in particular messenger RNA (mRNA); the synthetic forms of DNA or RNA; and polymers comprising a mixture of two or more of these molecules. The nucleic acid molecule may be linear or cyclic. Furthermore, the term "nucleic acid molecule" includes both sense and antisense strands, as well as single- and double-stranded forms. Moreover, the nucleic acid molecules described herein may contain naturally occurring or non-naturally occurring nucleotides. Examples of non-naturally occurring nucleotides include modified nucleotide bases having derived sugar or phosphate backbone linkages or chemically modified residues. The nucleic acid molecule also encompasses DNA and RNA molecules suitable for use as vectors for direct expression of the antibodies of the present disclosure *in vitro* and/or *in vivo,* e.g., in a host or patient. Such DNA (e.g., cDNA) or RNA (e.g., mRNA) vectors may be unmodified or modified. For example, mRNA may be chemically modified to enhance the stability of the RNA vector and/or the expression of the encoded molecule, so that the mRNA can be injected into a subject to produce antibodies *in vivo* (see, e.g., Stadler et al., Nature Medicine 2017, published online, June 12, 2017, doi: 10.1038/nm.4356 or EP 2 101 823 B1).

As used herein, the term "vector" includes nucleic acid vectors, e.g., DNA vectors (e.g., plasmids), RNA vectors, viruses, or other suitable replicons (e.g., viral vectors). Various vectors have been developed for the delivery of polynucleotides encoding foreign proteins into prokaryotic or eukaryotic cells. The expression vector of the present disclosure contains polynucleotide sequences as well as additional sequence elements, e.g., for expressing proteins and/or integrating these polynucleotide sequences into the genome of mammalian cells. Certain vectors that may be used to express the antibody and antibody fragment of the present disclosure include plasmids comprising regulatory sequences for directing gene transcription (e.g., promoter and enhancer regions). Other useful vectors for expressing the antibody and antibody fragment contain polynucleotide sequences that enhance the rate of translation of these genes or improve the stability or nuclear export of mRNA produced by gene transcription. These sequence elements include, for example, 5' and 3' untranslated regions, internal ribosome entry sites (IRESs), and polyadenylation signal sites, so as to direct the effective transcription of the gene carried on the expression vector. The expression vector of the present disclosure may also contain a polynucleotide encoding a marker for selecting cells harboring the vector. Examples of suitable markers include genes encoding resistance to antibiotics (e.g., ampicillin, chloramphenicol, kanamycin, or nourseothricin).

The term "host cell" herein refers to a cell into which an exogenous nucleic acid has been introduced, including the progeny of such a cell. Host cells include "transformants" and "transformed cells", which include primary transformed cells and progeny derived therefrom, regardless of the number of passages. The progeny may not be completely identical to the parent cells in terms of nucleic acid content, but may contain mutations. Mutant progeny that have the same function or biological activity as those screened or selected in the initially transformed cells are included herein.

The percent identity between two sequences varies with the identical positions shared by the sequences, taking into account the number of gaps that need to be introduced and the length of each gap for optimal alignment of the two sequences.

"n is a real number of 1-16" herein means that n is any real number greater than or equal to 1 and less than or equal to 16.

Herein, " 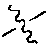 " represents a connection site.

The illustration method for the racemic or enantiomerically pure compounds herein is from Maehr, J. Chem. Ed. 1985, 62:114-120. Unless otherwise stated, the absolute configuration of a stereogenic center is represented by a wedged bond and a wedged dashed bond ( 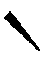 and 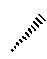 ), and the relative configuration of a stereogenic center (e.g., *cis-* or *trans*-configuration of alicyclic compounds) is represented by a black solid bond and a dashed bond ( 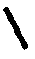 and 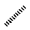 ).

The term "stereoisomer" refers to isomers resulting from different spatial arrangements of atoms in a molecule, including *cis-trans* isomers, enantiomers, and diastereoisomers.

The compound of the present disclosure may have an asymmetric atom such as a carbon atom, a sulfur atom, a nitrogen atom, and a phosphorus atom, or an asymmetric double bond, and thus the compound of the present disclosure may exist in the form of a particular geometric isomer or stereoisomer. The form of a particular geometric isomer or stereoisomer may be *cis* and *trans* isomers, *E* and *Z* geometric isomers, (-)- and (+)-enantiomers, (*R*)- and (*S*)-enantiomers, diastereoisomers, (*D*)-isomers, (*L*)-isomers, and racemic mixtures or other mixtures thereof, such as an enantiomer or diastereoisomer enriched mixture, and all of the above isomers, as well as mixtures thereof, are encompassed within the definition scope of the compound of the present disclosure. An additional asymmetric carbon atom, asymmetric sulfur atom, asymmetric nitrogen atom, or asymmetric phosphorus atom may be present in substituents such as alkyl. All of these isomers and mixtures thereof involved in the substituents are also encompassed within the definition scope of the compound of the present disclosure. The compound containing an asymmetric atom of the present disclosure can be separated in an optically active pure form or in a racemic form. The optically active pure form can be obtained by resolving a racemic mixture or by synthesis using chiral starting materials or chiral reagents.

The term "substituted" means that any one or more hydrogen atoms on a specific atom are replaced by substituents, as long as the valence of the specific atom is normal and the compound resulting from the substitution is stable. When the substituent is oxo (namely =O), it means that two hydrogen atoms are replaced, and oxo does not occur on an aromatic group.

The term "optional" or "optionally" means that the subsequently described event or circumstance may, but not necessarily, occur. The description includes instances where the event or circumstance occurs and instances where the event or circumstance does not occur. For example, ethyl being "optionally" substituted with halogen means that the ethyl may be unsubstituted (CH₂CH₃), monosubstituted (CH₂CH₂F, CH₂CH₂Cl, or the like), polysubstituted (CHFCH₂F, CH₂CHF₂, CHFCH₂Cl, CH₂CHCl₂, or the like), or fully substituted (CF₂CF₃, CF₂CCl₃, CCl₂CCl₃, or the like). It will be understood by those skilled in the art that for any group comprising one or more substituents, no substitution or substituting pattern that is spatially impossible and/or cannot be synthesized will be introduced.

Cₘ-Cₙ herein means having an integer number of carbon atoms within the range of m to n.

The term "alkyl" refers to a hydrocarbon group with a general formula of CnH₂ₙ₊₁. The alkyl may be linear or branched. The term "C₁-C₆ alkyl" should be understood to represent a linear or branched saturated hydrocarbon group having 1, 2, 3, 4, 5, or 6 carbon atoms. The alkyl includes, but is not limited to, methyl, ethyl, propyl, butyl, pentyl, hexyl, isopropyl, isobutyl, *sec-*butyl*, tert-*butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, and the like. The term "C₁-C₃ alkyl" refers to an alkyl group containing 1 to 3 carbon atoms, such as methyl, ethyl, *n*-propyl, or isopropyl.

The "C₁-C₆ alkyl" described herein may further include "C₁-C₃ alkyl".

The term "cycloalkyl" refers to a fully saturated carbon ring that exists in the form of a monocyclic ring, a fused ring, a bridged ring, a spiro ring, or the like. The term "C₃-C₆ cycloalkyl" should be understood to represent a saturated monocyclic, fused, spiro, or bridged ring having 3-6 carbon atoms. Specific examples include, but are not limited to, cyclopropyl, cyclobutyl, cyclopentyl, cyclohexyl, and the like.

The term "heterocyclyl" refers to a fully saturated or partially saturated monocyclic, fused cyclic, spirocyclic, or bridged cyclic group, and ring atoms of the group include 1-5 heteroatoms or heteroatom groups (i.e., heteroatom-containing atom groups). The "heteroatom or heteroatom group" includes, but is not limited to, a nitrogen atom (N), an oxygen atom (O), a sulfur atom (S), a phosphorus atom (P), a boron atom (B), -S(=O)2-, -S(=O)-, -P(=O)2-, -P(=O)-, -NH-, - S(=O)(=NH)-, -C(=O)NH-, -NHC(=O)NH-, or the like. The term "4- to 7-membered heterocyclyl" refers to a heterocyclyl group having 4, 5, 6, or 7 ring atoms, wherein 1-3 ring atoms are independently selected from the heteroatoms and heteroatom groups described above. The term "5- to 6-membered heterocyclyl" refers to a heterocyclyl group having 5 or 6 ring atoms, wherein 1-3 ring atoms are independently selected from the heteroatoms and heteroatom groups described above. Examples of 4-membered heterocyclyl include, but are not limited to, azetidinyl or oxetanyl; examples of 5-membered heterocyclyl include, but are not limited to, tetrahydrofuranyl, dioxolyl, pyrrolidinyl, imidazolidinyl, pyrazolidinyl, pyrrolinyl, 4,5-dihydrooxazolyl, or 2,5-dihydro-1*H*-pyrrolyl; examples of 6-membered heterocyclyl include, but are not limited to, tetrahydropyranyl, piperidinyl, morpholinyl, dithianyl, thiomorpholinyl, piperazinyl, trithianyl, tetrahydropyridinyl, or 4*H*-[1,3,4]thiadiazinyl; examples of 7-membered heterocyclyl include, but are not limited to, diazepanyl. "4- to 7-membered heterocyclyl" may include the ranges of "4- to 7-membered heterocycloalkyl", "5- to 6-membered heterocyclyl", "5- to 6-membered heterocycloalkyl", and the like.

The term "halo-" or "halogen" refers to fluorine, chlorine, bromine, and iodine.

The term "treatment" refers to surgical or therapeutic treatment for the purpose of preventing or slowing (reducing) the progression of an undesired physiological or pathological change, e.g., cancer, an autoimmune disease, and virus infection, in a subject being treated. Beneficial or desired clinical outcomes include, but are not limited to, alleviation of symptoms, decrease in disease severity, stabilization (i.e., not worsening) of disease state, delay or slowing of disease progression, amelioration or palliation of disease state, and remission (whether partial remission or complete remission) of disease state, whether detectable or undetectable. Subjects in need of treatment include those already suffering from a disorder or disease, as well as those prone to developing a disorder or disease or those intending to prevent a disorder or disease. When terms such as slowing, alleviation, decrease, palliation, and remission are referred to, their meanings also include elimination, disappearance, nonoccurrence, etc.

The term "effective amount" refers to an amount of a therapeutic agent that, when administered alone or in combination with another therapeutic agent to a cell, tissue or subject, is effective to prevent or alleviate symptoms of a disease or the progression of the disease. "Effective amount" also refers to an amount of a compound that is sufficient to alleviate symptoms, e.g., to treat, cure, prevent, or alleviate related medical disorders, or to increase the rates at which such disorders are treated, cured, prevented, or alleviated. When the active ingredient is administered alone to an individual, a therapeutically effective dose refers solely to the amount of the ingredient. When a combination is used, a therapeutically effective dose refers to the combined amount of the active ingredients that produces a therapeutic effect, regardless of whether they are administered in combination, sequentially, or simultaneously.

The term "subject" refers to an organism that receives treatment for a particular disease or disorder as described in the present disclosure. Examples of subjects and patients include mammals, such as humans, primates (e.g., monkeys), or non-primate mammals, that receive treatment for a disease or disorder.

The amount of the compound of the present disclosure constituting the "therapeutically effective amount" varies depending on the compound, the disease state and its severity, the route of administration, and the age of the mammal to be treated, but can be determined routinely by those skilled in the art in accordance with their knowledge and the present disclosure.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to salts of pharmaceutically acceptable acids or bases, including salts formed from the compound and an inorganic or organic acid, and salts formed from the compound and an inorganic or organic base.

The term "pharmaceutical composition" refers to a mixture consisting of one or more of the compounds or the salts thereof of the present disclosure and a pharmaceutically acceptable excipient. The pharmaceutical composition is intended to facilitate the administration of the compound of the present disclosure to an organism.

The term "pharmaceutically acceptable excipient" refers to those excipients that exhibit no significant irritating effect on an organism and do not impair the biological activity and properties of the active compound. Suitable excipients are well known to those skilled in the art, such as carbohydrates, waxes, water-soluble and/or water-swellable polymers, hydrophilic or hydrophobic materials, gelatin, oils, solvents, water, and the like.

The word "comprise" and variations thereof such as "comprises" or "comprising" may be understood in an open and non-exclusive sense, i.e., "including but not limited to".

The present disclosure also includes isotopically labeled compounds of the present disclosure which are identical to those documented herein but have one or more atoms replaced by an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, ³⁶Cl, respectively, and the like.

Certain isotopically labeled compounds of the present disclosure (e.g., those labeled with ³H and ¹⁴C) can be used for compound and/or substrate tissue distribution analysis. Tritiated (i.e., ³H) and carbon-14 (i.e., ¹⁴C) isotopes are particularly preferred due to their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present disclosure can generally be prepared by following procedures analogous to those disclosed in the schemes and/or examples below while substituting a non-isotopically labeled reagent with an isotopically labeled reagent.

The pharmaceutical composition of the present disclosure may be suitable for parenteral administration, such as a sterile solution, suspension or lyophilized product in a suitable unit dosage form. For example, the pharmaceutical composition of the present disclosure may be in the form of a sterile injectable aqueous solution for intramuscular or subcutaneous administration. Other vehicles or solvents such as water, Ringer's solution or isotonic sodium chloride solution are acceptable when the pharmaceutical composition of the present disclosure is used.

In all of the administration methods for the compound described herein, the daily dose administered is from 0.001 mg/kg body weight to 600 mg/kg body weight, preferably from 0.05 mg/kg body weight to 200 mg/kg body weight, and more preferably from 0.1 mg/kg body weight to 100 mg/kg body weight, given in a single dose or divided doses.

The compounds of the present disclosure can be prepared by a variety of synthetic methods well known to those skilled in the art, including the specific embodiments listed below, embodiments formed by combinations thereof with other chemical synthetic methods, and equivalents thereof well known to those skilled in the art. The preferred embodiments include, but are not limited to, the examples of the present disclosure.

The chemical reactions of the specific embodiments of the present disclosure are conducted in a suitable solvent that must be suitable for the chemical changes in the present disclosure and the reagents and materials required. In order to obtain the compounds of the present disclosure, it is sometimes necessary for those skilled in the art to modify or select a synthetic procedure or a reaction process based on the existing embodiments.

An important consideration in synthetic route planning in the art is the selection of suitable protecting groups for reactive functional groups (e.g., amino and carboxyl in the present disclosure). For example, reference may be made to Greene's Protective Groups in Organic Synthesis (4th Ed.) Hoboken, New Jersey: John Wiley & Sons, Inc. All references cited in the present disclosure are incorporated herein in their entirety.

### BRIEF DESCRIPTION OF THE DRAWINGS

Unless otherwise defined herein, scientific and technical terms used in correlation with the present disclosure shall have the meanings that are commonly understood by those skilled in the art.
FIG. 1 shows the results of X-ray single-crystal diffraction analysis of compound 19-P1.
FIG. 2 shows the inhibitory effects of ADCs on OVCAR3 cell proliferation.
FIG. 3 shows the inhibitory effects of ADCs on PA-1 cell proliferation.
FIG. 4 shows the inhibitory effects of ADCs on OVCAR3 cell proliferation.
FIG. 5 shows the inhibitory effects of ADCs on PA-1 cell proliferation.
FIG. 6 shows the tumor growth curves of the OVCAR3 subcutaneous tumor model.
FIG. 7 shows the tumor growth curves of the OVCAR3 subcutaneous tumor model.
FIG. 8 shows the tumor growth curves of the PA-1 subcutaneous tumor model.
FIG. 9 shows the tumor growth curves of the PA-1 subcutaneous tumor model.
FIG. 10 shows the tumor growth curves of the 786-O subcutaneous tumor model.

### DETAILED DESCRIPTION

The present disclosure will be further described with reference to specific examples, and the advantages and features of the present disclosure will become more apparent with the description. Experimental procedures without specified conditions in the examples are conducted according to conventional conditions or conditions recommended by the manufacturers. Reagents or instruments without specified manufacturers used herein are conventional products that are commercially available.

The examples are exemplary only and do not limit the scope of the present disclosure in any way. It will be understood by those skilled in the art that various changes or substitutions in form and details may be made to the technical solutions of the present disclosure without departing from the spirit and scope of the present disclosure, and that these changes and substitutions shall fall within the protection scope of the present disclosure.

### Example 1-1: Synthesis of 2-cyclopropyl-N-(((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)-2-hydroxyacetamide (compound 14) and isomers 14-P1 and 14-P2

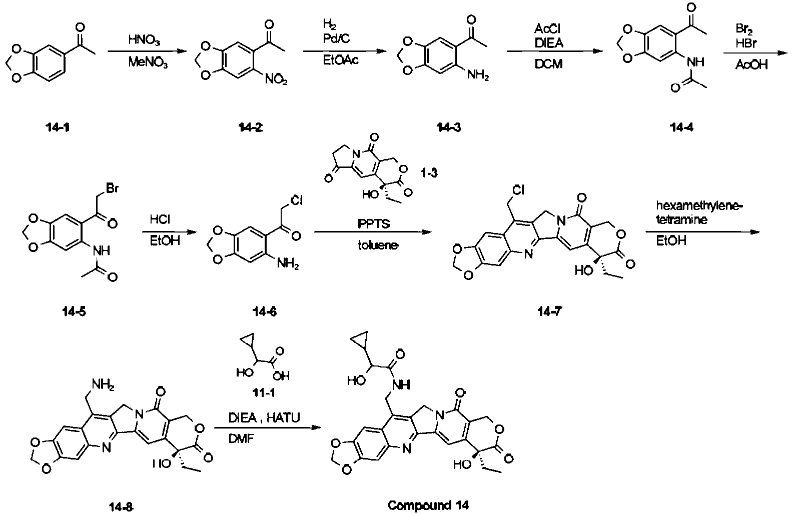

### Step 1: Synthesis of 1-(6-nitrobenzo[d][1,3]dioxol-5-yl)ethanone (intermediate 14-2)

Intermediate 14-1 (10.0 g, 60.92 mmol) was dissolved in nitromethane (100 mL), nitric acid (35.43 g, 365.50 mmol, 65% purity) was slowly added thereto, and the reaction mixture was stirred at 25 °C for 2.5 h. After the reaction was completed, a saturated sodium bicarbonate solution was slowly added to the reaction mixture to adjust the pH to 7-8, and dichloromethane (100 mL) was then added thereto. The organic phase was washed with water (50 mL × 2), and the washed organic phase was dried over an appropriate amount of anhydrous sodium sulfate. The residue was purified by preparative thin-layer chromatography (petroleum ether:ethyl acetate = 1:2) to give the title compound (5 g).

MS m/z (ESI): 210.0 [M+H]+.

### Step 2: Synthesis of 1-(6-aminobenzo[d][1,3]dioxol-5-yl)ethanone (intermediate 14-3)

Intermediate 14-2 (2.37 g, 11.33 mmol) was dissolved in absolute ethanol (25 mL), palladium on carbon (0.2 g, 10% purity) was added thereto, and the reaction mixture was stirred at 25 °C for 16 h under a hydrogen atmosphere. After the reaction was completed, the reaction mixture was filtered, the filter cake was washed twice with ethyl acetate, and the filtrate was concentrated to dryness under reduced pressure to give the title compound (1.6 g).

MS m/z (ESI): 180.1 [M+H]+.

### Step 3: Synthesis of N-(6-acetylbenzo[d][1,3]dioxol-5-yl)acetamide (intermediate 14-4)

Intermediate 14-3 (1.0 g, 5.58 mmol) was dissolved in dichloromethane (10 mL), the reaction mixture was cooled to 0 °C, and *N,N*-diisopropylethylamine (DIEA) (1.08 g, 8.37 mmol) and acetyl chloride (569.55 mg, 7.26 mmol) were added thereto. The reaction mixture was stirred at 25 °C for 1.5 h. After the reaction was completed, the reaction mixture was concentrated to dryness under reduced pressure to give the title compound (1.23 g).

MS m/z (ESI): 222.1 [M+H]+.

### Step 4: Synthesis of N-(6-(2-bromoacetyl)benzo[d][1,3]dioxol-5-yl)acetamide (intermediate 14-5)

**Intermediate 14-4** (1.23 g, 5.00 mmol) was dissolved in acetic acid (12 mL), a solution of hydrogen bromide in acetic acid (1.84 g, 7.51 mmol, 33% purity) was added thereto, and then liquid bromine (959.69 mg, 6.01 mmol) was slowly added thereto. The reaction mixture was stirred at 25 °C for 1 h. After the reaction was completed, the reaction mixture was poured into ice water and stirred for 10 min. The mixture was then filtered. The filter cake was washed twice with water and concentrated to dryness under reduced pressure. Ethyl acetate (2 mL) and petroleum ether (10 mL) were added to the residue, and the resulting reaction mixture was stirred at 25 °C for 0.5 h. The reaction mixture was filtered, and the filter cake was dried to give the title compound (500 mg).

MS m/z (ESI): 300.0 [M+H]+.

### Step 5: Synthesis of 1-(6-aminobenzo[d][1,3]dioxol-5-yl)-2-chloroethanone (intermediate 14-6)

**Intermediate 14-5** (0.2 g, 666.43 µmol) was dissolved in absolute ethanol (1 mL) and concentrated hydrochloric acid (1 mL), and the reaction mixture was stirred at 60 °C for 16 h. After the reaction was completed, the reaction mixture was cooled to room temperature, ice water (10 mL) and saturated sodium bicarbonate (10 mL) were slowly and sequentially added, and then dichloromethane (50 mL) was added. The organic phase was washed with water (20 mL × 2), and the washed organic phase was dried over an appropriate amount of anhydrous sodium sulfate. The residue was subjected to preparative thin-layer chromatography (petroleum ether:ethyl acetate = 6:1) to give the title compound (160 mg).

MS m/z (ESI): 214.0 [M+H]+.

### Step 6: Synthesis of (S)-14-(bromomethyl)-7-ethyl-7-hydroxy-10,13-dihydro-11H-[1,3]dioxolo[4,5-g]pyrano[3',4':6,7]indolizino[1,2-b]quinolin-8,11(7H)-dione (intermediate 14-7)

**Intermediate 14-6** (50 mg, 234.06 µmol) and intermediate 1-3 (61.62 mg, 234.06 µmol) were dissolved in toluene (1 mL), and pyridinium *p*-toluenesulfonate (5.88 mg, 23.41 µmol) was added thereto. The reaction mixture was stirred at 90 °C for 16 h. After the reaction was completed, the reaction mixture was cooled to room temperature, ethanol (1 mL) was added, and the reaction mixture was stirred at 25 °C for 0.5 h. The reaction mixture was filtered, and the filter cake was washed with ethanol (2 mL × 2) and dried to give the title compound (60 mg).

MS m/z (ESI): 441.1 [M+H]+.

### Step 7: Synthesis of (S)-14-(aminomethyl)-7-ethyl-7-hydroxy-10,13-dihydro-11H-[1,3]dioxolo[4,5-g]pyrano[3',4':6,7]indolizino[1,2-b]quinolin-8,11(7H)-dione (intermediate 14-8)

**Intermediate 14-7** (55.00 mg, 124.76 µmol) was dissolved in ethanol (1 mL), and urotropin (52.47 mg, 374.29 µmol) was added thereto. The reaction mixture was stirred at 80 C for 1.5 h. After the reaction was completed, the reaction mixture was cooled to room temperature and concentrated to dryness under reduced pressure. The residue was purified by preparative high-performance liquid chromatography (YMC-Actus Triart C18 column, 5 µm, 25 mm in diameter, 100 mm in length; a mixture of water (containing 0.225% formic acid) and methanol with decreasing polarity was used as the eluent; methanol gradient: 0%-27%, elution time: 12 min) to give the title compound (10 mg).

MS m/z (ESI): 422.1 [M+H]+.

### Step 8: Synthesis of 2-cyclopropyl-N-(((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)-2-hydroxyacetamide (compound 14)

**Intermediate 14-8** (10.00 mg, 20.17 µmol) and intermediate 11-1 (23.42 mg, 201.71 µmol) were dissolved in anhydrous *N,N*-dimethylformamide (0.5 mL), and *O*-(7-azabenzotriazol-1-yl)-*N,N,N',N*'-tetramethyluronium hexafluorophosphate (HATU) (11.50 mg, 30.26 µmol) and *N,N-*diisopropylethylamine (7.82 mg, 60.51 µmol) were added thereto. The reaction mixture was stirred at 25 °C for 1.5 h. After the reaction was completed, the reaction mixture was filtered and purified by preparative high-performance liquid chromatography (YMC-Actus Triart C18 column, 5 µm, 30 mm in diameter, 150 mm in length; a mixture of water (containing 0.225% formic acid) and acetonitrile with decreasing polarity was used as the eluent; acetonitrile gradient: 4%-44%, elution time: 9 min) to give the title compound (3 mg).

MS m/z (ESI): 520.1 [M+H]+.

**1H NMR (400 MHz, DMSO-d6) δ** = 8.37 (t, J = 5.8 Hz, 1H), 7.62 (s, 1H), 7.28 (s, 1H), 7.00 (s, 1H), 6.25 (s, 1H), 6.05 (s, 2H), 5.27 (d, J = 5.0 Hz, 1H), 5.23 (s, 2H), 5.18 (s, 2H), 4.48 (d, J = 5.5 Hz, 2H), 1.66-1.55 (m, 2H), 0.76-0.40 (m, 1H), 0.63 (t, J = 7.3 Hz, 3H), 0.14-0.06 (m, 2H), 0.05-0.04 (m, 2H).

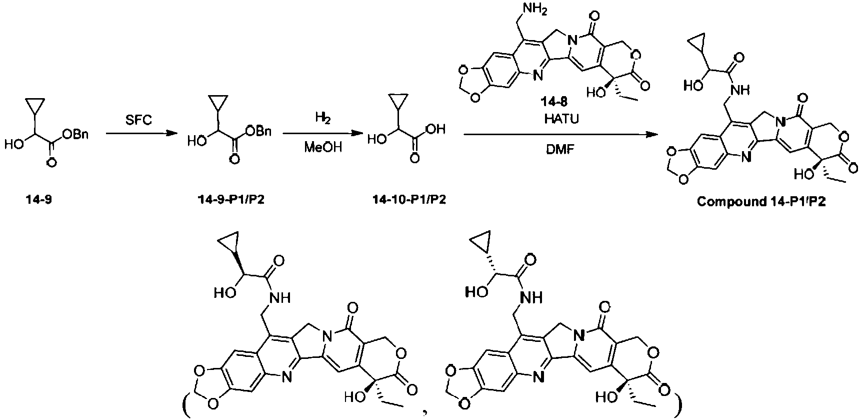

### Step 9: Preparation of benzyl 2-cyclopropyl-2-hydroxyacetate (intermediate 14-9-P1/P2)

Intermediate 14-9 was resolved to prepare isomers 14-9-P1 and 14-9-P2. Intermediate 14-9 (1.3 g) was subjected to preparative supercritical fluid chromatography (DAICEL CHIRALPAK AD column, 10 µm silica, 30 mm in diameter, 250 mm in length; ethanol (containing 0.1% ammonia water) was used as the eluent) to give intermediate 14-9-P1 (600 mg) and intermediate 14-9-P2 (600 mg).

The two isomers described above were further analyzed under the following chiral supercritical fluid chromatographic conditions.

| | | |
|---|---|---|
| Column | Chiralpak AD-3 150*4.6mm I.D., 3um | |
| Mobile phase | A: carbon dioxide | |
| | B: methanol (0.05% diethylamine) | |
| | Time (min) | Phase B% |
| | 0 | 5 |
| | 4 | 40 |
| | 0.2 | 5 |
| | 1.8 | 5 |
| Flow rate | 2.5 mL/min | |
| Wavelength | PDA 220nm | |
| Column temperature | 35°C | |
| Column pressure | 1500 Psi | |
| Instrument model | Waters UPCC with PDA Detector | |

### Intermediate 14-9-P1:

Under the chiral supercritical fluid chromatographic conditions described above, the retention time was 2.990 min.

**¹H NMR (400 MHz, METHANOL-*d*₄)** δ 7.43-7.29 (m, 5H), 5.29-5.16 (m, 2H), 3.67 (d, *J* = 7.6 Hz, 1H), 1.19-1.07 (m, 1H), 0.58-0.38 (m, 4H).

### Intermediate 14-9-P2:

Under the chiral supercritical fluid chromatographic conditions described above, the retention time was 2.661 min.

**¹H NMR (400 MHz, METHANOL-*d*₄)** δ 7.46-7.28 (m, 5H), 5.30-5.16 (m, 2H), 3.67 (d, *J* = 7.6 Hz, 1H), 1.21-1.03 (m, 1H), 0.60-0.36 (m, 4H).

### Step 10: Synthesis of 2-cyclopropyl-2-hydroxyacetic acid (intermediate 14-10-P1/P2)

**Intermediate 14-9-P1** (500 mg) was added to methanol (15 mL) under a hydrogen atmosphere, wet palladium on carbon (10 mg, 10%) was added to the reaction mixture, and the reaction mixture was stirred at 25 °C for 16 h under a hydrogen atmosphere. After the reaction was completed, the reactant was filtered, and the filtrate was concentrated under reduced pressure to give **intermediate 14-10-P1** (273 mg).

**¹H NMR (400 MHz, METHANOL-*d*₄)** δ 3.63 (d, *J* = 7.2 Hz, 1H), 1.21-1.09 (m, 1H), 0.61-0.40 (m, 4H).

**Intermediate 14-9-P2** (500 mg) was added to methanol (15 mL) under a hydrogen atmosphere, wet palladium on carbon (10 mg, 10%) was added to the reaction mixture, and the reaction mixture was stirred at 25 °C for 16 h under a hydrogen atmosphere. After the reaction was completed, the reactant was filtered, and the filtrate was concentrated under reduced pressure to give **intermediate 14-10-P2** (279 mg).

**¹H NMR (400 MHz, METHANOL-*d*₄)** δ 3.63 (d, *J* = 7.2 Hz, 1H), 1.19-1.08 (m, 1H), 0.60-0.39 (m, 4H).

### Step 11: Synthesis of 2-cyclopropyl-N-(((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)-2-hydroxyacetamide (compound 14-P1/P2)

**Intermediate 14-8** (40.00 mg) and **intermediate 14-10-P1** (28.11 mg) were dissolved in anhydrous *N,N*-dimethylformamide (1 mL), and *O*-(7-azabenzotriazol-1-yl)-*N,N,N',N*'-tetramethyluronium hexafluorophosphate (HATU) (46.02 mg) and *N,N*-diisopropylethylamine (31.28 mg) were added thereto. The reaction mixture was stirred at 25 °C for 1.5 h. After the reaction was completed, the reaction mixture was purified by preparative high-performance liquid chromatography (Boston Green ODS C18 column, 5 µm silica, 30 mm in diameter, 150 mm in length; a mixture of water (containing 0.225% formic acid) and acetonitrile with decreasing polarity was used as the eluent (acetonitrile gradient: 16%-46%, elution time: 12 min) to give **compound 14-P1** (22.00 mg).

MS m/z (ESI): 520.1 [M+H]⁺.

**¹H NMR (400 MHz, DMSO-*d*₆)** δ = 8.62 (t, *J =* 5.7 Hz, 1H), 7.86 (s, 1H), 7.52 (s, 1H), 7.25 (s, 1H), 6.51 (s, 1H), 6.29 (s, 2H), 5.47 (s, 2H), 5.43 (s, 2H), 4.73 (d, *J =* 5.9 Hz, 2H), 3.54 (d, *J* = 5.9 Hz, 1H), 1.93-1.78 (m, 2H), 1.06-0.96 (m, 1H), 0.87 (t, *J =* 7.3 Hz, 3H), 0.39-0.30 (m, 2H), 0.29-0.21 (m, 2H).

**Intermediate 14-8** (10.00 mg) and **intermediate 14-10-P2** (8.27 mg) were dissolved in anhydrous *N,N*-dimethylformamide (0.5 mL), and *O*-(7-azabenzotriazol-1-yl)-*N,N,N',N'-*tetramethyluronium hexafluorophosphate (18.05 mg) and N,N-diisopropylethylamine (6.13 mg) were added thereto. The reaction mixture was stirred at 25 °C for 1.5 h. After the reaction was completed, the reaction mixture was directly purified by preparative high-performance liquid chromatography (Boston Green ODS C18 column, 5 µm silica, 30 mm in diameter, 150 mm in length; a mixture of water (containing 0.225% formic acid) and acetonitrile with decreasing polarity was used as the eluent (acetonitrile gradient: 16%-46%, elution time: 12 min) to give **compound 14-P2** (8.00 mg).

MS m/z (ESI): 520.1 [M+H]⁺.

**¹H NMR (400 MHz, DMSO-*d*₆)** δ = 8.63 (t, *J =* 5.9 Hz, 1H), 7.86 (s, 1H), 7.52 (s, 1H), 7.24 (s, 1H), 6.30 (s, 2H), 5.46 (s, 2H), 5.43 (s, 2H), 4.72 (d, *J =* 6.0 Hz, 2H), 3.55 (d, *J =* 6.0 Hz, 1H), 1.92-1.81 (m, 2H), 1.03-0.97 (m, 1H), 0.88 (t, *J=* 7.3 Hz, 3H), 0.38-0.30 (m, 2H), 0.28-0.22 (m, 2H).

The two isomers were separately further analyzed by the following chiral supercritical fluid chromatography analysis method.

| | |
|---|---|
| Column | Chiralcel OJ-3 100A 4.6mm I.D., 3µm |
| Mobile phase | A: carbon dioxide |
| | B: ethanol (0.05% diethylamine) |
| Flow rate | 2.8 mL/min |
| Wavelength | PDA 254nm |
| Column temperature | 35 °C |
| Column pressure | 1500 Psi |
| Instrument model | Waters UPCC with PDA Detector and QDa Detector |

### Compound 14-P1:

Under the chiral supercritical fluid chromatographic conditions described above, the retention time was 3.673 min.

### Compound 14-P2:

Under the chiral supercritical fluid chromatographic conditions described above, the retention time was 3.735 min.

### Example 1-2: (S)-2-Cyclopropyl-N-(((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)-2-hydroxyacetamide (compound 14-S)

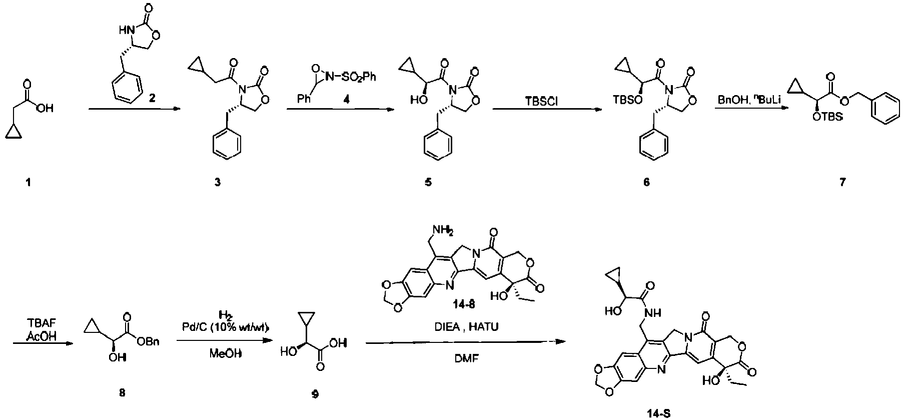

### Step 1: Synthesis of (S)-4-benzyl-3-(2-cyclopropylacetyl)oxazolidin-2-one (intermediate 3)

**Starting material 1** (150.0 g), 4-dimethylaminopyridine (160.15 g), and starting material 2 (221.2 g) were weighed out and dissolved in 1500 mL of dichloromethane, and the mixture was stirred at room temperature for 15 min. 1-Ethyl-(3-dimethylaminopropyl)carbodiimide hydrochloride (EDCI, 359.0 g) was weighed out and added to the reaction mixture in batches. After the addition was completed, the resulting mixture was stirred at room temperature for about 5 h. After the reaction was completed, dichloromethane (1500 mL) was added to the reaction mixture for dilution. The mixture was then sequentially washed twice with water (500 mL), once with 2 N HCl (500 mL), once with a saturated sodium bicarbonate solution (500 mL) and once with a saturated brine solution (500 mL). The organic phase was dried over anhydrous sodium sulfate and filtered. The filtrate was concentrated to dryness under reduced pressure to give the title compound (302 g).

**¹H NMR (400 MHz, CDCl₃)** δ 7.32-7.35 (m, 2H), 7.26-7.29 (m, 1H), 7.21-7.23 (m, 2H), 4.68-4.71 (m, 1H), 4.17-4.23 (m, 2H), 3.30-3.33 (dd, 1H), 2.91-2.95 (dd, 1H), 2.78-2.82 (m, 2H), 1.14-1.18 (m, 1H), 0.59-0.63 (m, 2H), 0.21-0.26 (m, 2H).

### Step 2: Synthesis of (S)-4-benzyl-3-((S)-2-cyclopropyl-2-hydroxyacetyl)oxazolidin-2-one (intermediate 5)

**Intermediate 3** (200 g) was weighed out and dissolved in 2000 mL of anhydrous tetrahydrofuran, and the mixture was stirred at -78 °C for 15 min under a nitrogen atmosphere. Subsequently, sodium bis(trimethylsilyl)amide (443.5 mL, 2 M in tetrahydrofuran) was added dropwise to the reaction mixture. After the dropwise addition was completed, the reaction mixture was stirred at -78 °C for 30 min. **Intermediate 4** (201.5 g) was completely dissolved in 700 mL of tetrahydrofuran, and the resulting solution was added dropwise and slowly to the reaction mixture. After the dropwise addition was completed, the mixture was stirred at -78 °C for 2 h. Subsequently, 220 mL of glacial acetic acid was added to the reaction mixture to quench the reaction. After the dropwise addition was completed, the mixture was gradually warmed to room temperature, 600 mL of 2 N HCl was added to the reaction mixture, and the resulting mixture was stirred at room temperature (20-25°C) for 10 h. Subsequently, the reaction mixture was concentrated under reduced pressure, ethyl acetate (1000 mL) and water (200 mL) were added to the residue, and the mixture was stirred for 20 min. The aqueous phase was separated and then extracted twice with ethyl acetate (500 mL × 2). The organic phases were combined, and sequentially washed twice with 400 mL of a saturated NaHCO₃ solution, twice with 400 mL of a saturated Na₂S₂O₃ solution and twice with saturated brine. The resulting organic phase was dried over anhydrous sodium sulfate and filtered. The filtrate was then concentrated to dryness under reduced pressure, and the residue was purified by silica gel column chromatography (petroleum ether:ethyl acetate = 1/30) to give the title compound (128.7 g).

**¹H NMR (400 MHz, CDCl₃)** δ 7.37 (dd, J = 8.1, 6.8 Hz, 2H), 7.33-7.29 (m, 1H), 7.27-7.23 (m, 2H), 4.81 (dd, J = 7.9, 5.9 Hz, 1H), 4.72 (ddt, J = 10.0, 7.5, 2.9 Hz, 1H), 4.33 (t, J = 8.3Hz, 1H), 4.28 (dd, J = 9.1, 2.5 Hz, 1H), 3.48 (dd, J = 8.2, 3.9 Hz, 1H), 3.35 (dd, J = 13.5, 3.4 Hz,1H), 2.88 (dd, J = 13.5, 9.4 Hz, 1H), 1.36-1.29 (m, 1H), 0.62-0.44 (m, 4H).

### Step 3: Synthesis of (S)-4-benzyl-3-((S)-2-((tert-butyldimethylsilyl)oxy)-2-cyclopropylacetyl) oxazolidin-2-one (intermediate 6)

**Intermediate 5** (128.7 g) was weighed out and dissolved in 1300 mL of dichloromethane. Imidazole (56.17 g) was added, and the mixture was stirred for 15 min under an ice bath. TBSCl (107.3 g) was then added to the reaction mixture in batches, and the resulting mixture was stirred at room temperature for 3 h. 200 mL of 2 N HCl was added to the reaction mixture, and the resulting mixture was stirred for 20 min, followed by liquid separation. The organic phase was sequentially washed twice with 200 mL of a saturated NaHCO₃ solution and twice with saturated brine. The organic phase was dried over anhydrous sodium sulfate and filtered. The filtrate was then concentrated to dryness under reduced pressure, and the residue was purified by silica gel column chromatography (petroleum ether:ethyl acetate = 80:1) to give the title compound (160 g).

**¹H NMR (400 MHz, CDCl₃)** δ 7.35-7.37 (m, 2H), 7.30-7.32 (m, 1H), 7.26-7.29 (m, 2H), 5.26-5.31 (m, 1H), 4.67-4.71 (m, 1H), 4.20-4.26 (m, 2H), 3.41-3.44 (dd, 1H), 2.72-2.76 (dd, 1H), 1.25-1.31 (m, 1H), 0.94 (s, 9H), 0.54-0.56 (m, 2H), 0.46-0.48 (m, 2H), 0.12 (s, 6H).

### Step 4: Synthesis of benzyl (S)-2-((tert-butyldimethylsilyl)oxy)-2-cyclopropylacetate (intermediate 7)

Benzyl alcohol (62.18 g) was weighed out and dissolved in 500 mL of tetrahydrofuran, and the mixture was stirred at -25 °C. *n*-Butyllithium (213.6 mL, 2.5 M in tetrahydrofuran) was measured out and added dropwise and slowly to the reaction mixture. After the dropwise addition was completed, the mixture was stirred at -25 °C for 1 h. **Intermediate 6** (160 g) was weighed out and dissolved in 320 mL of tetrahydrofuran, and the resulting solution was added dropwise and slowly to the reaction mixture at -25 °C. After the dropwise addition was completed, the mixture was stirred at -15 °C for 3 h. A saturated NH4Cl solution (200 mL) was added to the reaction mixture to quench the reaction. The reaction mixture was then concentrated under reduced pressure, and 400 mL of methyl *tert-*butyl ether and water (150 mL) were added to the reaction mixture. The resulting mixture was stirred for 30 min, followed by liquid separation. The aqueous phase was extracted twice with methyl *tert-*butyl ether (200 mL × 2). The organic phases were combined, washed once with saturated brine (250 mL), dried over anhydrous sodium sulfate, and filtered. The filtrate was then concentrated under reduced pressure, and the residue was purified by silica gel column chromatography (petroleum ether:ethyl acetate = 100:1) to give the title compound (125 g).

**¹H NMR (400 MHz, CDCl₃)** δ 7.28-7.40 (m, 5H), 5.17-5.26 (m, 2H), 3.86-3.89 (m, 1H), 1.21-1.46 (m, 1H), 0.90 (s, 9H), 0.45-0.51 (m, 4H), 0.05 (s, 6H).

### Step 5: Synthesis of benzyl (S)-2-cyclopropyl-2-hydroxyacetate (intermediate 8)

**Intermediate 7** (125 g) was weighed out and dissolved in 1200 mL of tetrahydrofuran. Glacial acetic acid (35.1 g) was added, and the mixture was stirred at room temperature for 5 min. Tetrabutylammonium fluoride (TBAF, 585 mL, 1 M in tetrahydrofuran) was then added to the reaction mixture, and the reaction mixture was allowed to react at 45 °C for 4 h. The reaction mixture was concentrated under reduced pressure to remove tetrahydrofuran (600 mL), and 300 mL of water and 400 mL of methyl *tert-*butyl ether were added to the residue. The mixture was stirred for 20 min, followed by liquid separation. The aqueous phase was extracted twice with methyl *tert-*butyl ether (200 mL). The organic phases were combined, sequentially washed twice with 200 mL of a saturated NaHCO₃ solution and twice with saturated brine, then dried over anhydrous sodium sulfate, and filtered. The filtrate was concentrated to dryness under reduced pressure, and the residue was purified by silica gel column chromatography (petroleum ether:ethyl acetate = 60:1) to give the title compound (68.9 g).

**¹H NMR (400 MHz, CDCl₃)** δ 7.27-7.39 (m, 5H), 5.22-5.28 (m, 2H), 3.82 (d, 1H), 2.7 (brs, 1H), 1.11-1.15 (m, 1H), 0.41-0.56 (m, 4H).

**Intermediate 8** was further analyzed under the following chiral supercritical fluid chromatographic conditions.

| | | |
|---|---|---|
| Column | Chiralpak AD-3 150*4.6mm I.D., 3um | |
| Mobile phase | A: carbon dioxide | |
| | B: methanol (0.05% diethylamine) | |
| | Time (min) | Phase B% |
| | 0 | 5 |
| | 4 | 40 |
| | 0.2 | 5 |
| | 1.8 | 5 |
| Flow rate | 2.5 mL/min | |
| Wavelength | PDA 220nm | |
| Column temperature | 35°C | |
| Column pressure | 1500 Psi | |
| Instrument model | Waters UPCC with PDA Detector | |

Under the chiral supercritical fluid chromatographic conditions described above, the retention time of **intermediate 8** was 3.013 min, which was substantially consistent with the retention time (2.990 min) of **intermediate 14-9-P1** in Example 1-1 under the same chromatographic analysis conditions. **Intermediate 8** has the same configuration as **intermediate 14-9-P1,** both being the same compound.

### Step 6: Synthesis of (S)-2-cyclopropyl-2-hydroxyacetic acid (intermediate 9)

**Intermediate 8** (5 g) was dissolved in methanol (80 mL), wet palladium on carbon (10% mass content, 0.7 g) was added to the reaction mixture, and the resulting mixture was stirred at 25 °C for 16 h under a hydrogen atmosphere. After the reaction was completed, the reaction mixture was filtered, and the filtrate was concentrated to dryness under reduced pressure to give the title compound (2.4 g).

**¹H NMR (400 MHz, METHANOL-*d*₄)** δ = 3.63 (d, *J =* 7.3 Hz, 1H), 1.20-1.09 (m, 1H), 0.61-0.39 (m, 4H).

### Step 7: Synthesis of (S)-2-cyclopropyl-N-(((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)methyl)-2-hydroxyacetamide (compound 14-S)

**Intermediate 14-8** (90 mg) and **intermediate 9** (49.60 mg) were dissolved in anhydrous *N,N-*dimethylformamide (1 mL), and *O*-(7-azabenzotriazol-1-yl)-*N,N,N,N*-tetramethyluronium hexafluorophosphate (121.81 mg) and *N,N*-diisopropylethylamine (82.81 mg) were added thereto. The reaction mixture was stirred at 25 °C for 16 h. After the reaction was completed, the reaction mixture was purified by high-performance liquid chromatography (column: Boston Green ODS 150 × 30 mm × 5 µm; mobile phase: [A: water (0.225% formic acid), B: acetonitrile]; B%: 20%-50%, 12 min) to give the title compound (21 mg).

MS m/z (ESI): 520.0 [M+H]⁺.

**¹H NMR (400 MHz, DMSO-*d*₆)** δ = 8.62 (t, *J =* 6.1 Hz, 1H), 7.87 (s, 1H), 7.52 (s, 1H), 7.24 (s, 1H), 6.48 (s, 1H), 6.30 (s, 2H), 5.48 (s, 2H), 5.43 (s, 2H), 4.73 (d, *J* =5.6 Hz, 2H), 3.54 (d, *J =* 5.5 Hz, 1H), 1.93-1.80 (m, 2H), 1.05-0.97 (m, 1H), 0.88 (t, *J =* 7.3 Hz, 3H), 0.39-0.30 (m, 2H), 0.30-0.22 (m, 2H).

**Compound 14-S** was further analyzed under the following chiral supercritical fluid chromatographic conditions.

| | |
|---|---|
| Column | Chiralcel OJ-3 100A 4.6mm I.D., 3µm |
| Mobile phase | A: carbon dioxide |
| | B: ethanol (0.05% diethylamine) |
| Flow rate | 2.8 mL/min |
| Wavelength | PDA 254nm |
| Column temperature | 35°C |
| Column pressure | 1500 Psi |
| Instrument model | Waters UPCC with PDA Detector and QDa Detector |

Under the chiral supercritical fluid chromatographic conditions described above, the retention time of **compound 14-S** was 3.654 min, which was substantially consistent with the retention time (3.673 min) of **compound 14-P1** prepared in Example 1-1 under the same chromatographic analysis conditions. Thus, **compound 14-S** was determined to have the same configuration as **compound 14-P1** prepared in Example 1-1, both being the same compound.

### Example 2-1: Synthesis of 2-cyclopropyl-N-(((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)methyl)-2-hydroxyacetamide (compound 19) and isomers 19-P1 and 19-P2

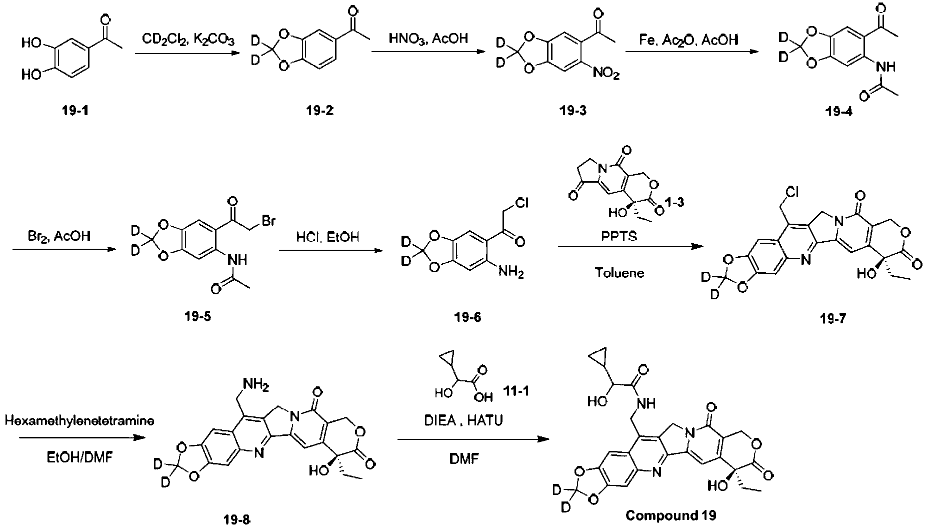

### Step 1: Synthesis of 1-(benzo[d][1,3]dioxol-5-yl-2,2-d₂)ethane-1-one (intermediate 19-2)

**Intermediate 19-1** (3 g) was dissolved in an anhydrous DMF solution (25 mL), and deuterated dichloromethane (8.57 g) and potassium carbonate (8.18 g) were added. After the addition was completed, the mixture was warmed to 90 °C and stirred for 16 h. The reaction mixture was then added to water (100 mL), and the resulting mixture was extracted with ethyl acetate (200 mL × 2). The organic phases were combined, washed with saturated brine (100 mL), dried over anhydrous sodium sulfate, and filtered. The filtrate was concentrated to dryness under reduced pressure, and the residue was purified by column chromatography (ethyl acetate/petroleum ether = 5:1) to give the title compound (2.4 g).

MS m/z (ESI): 167.1 [M+H]⁺.

### Step 2: Synthesis of 1-(6-nitrobenzo[d][1,3]dioxol-5-yl-2,2-d₂)ethane-1-one (intermediate 19-3)

**Intermediate 19-2** (2.4 g) was dissolved in anhydrous acetic acid (10 mL), and concentrated nitric acid (32.50 g, 70% content) was added dropwise at 0 °C. After the addition was completed, the mixture was stirred at 0 °C for 10 min. The mixture was then warmed to room temperature and stirred for 1 h. After the reaction was completed, the reaction mixture was added dropwise to ice water (200 mL). The resulting mixture was filtered, and the filter cake was dried to give the title compound (1.9 g).

MS m/z (ESI): 212.0 [M+H]⁺.

**¹H NMR (400 MHz, DMSO-*d*₆)** δ 7.69 (s, 1H), 7.30 (s, 1H), 2.49 (s, 3H).

### Step 3: Synthesis of N-(6-acetylbenzo[d][1,3]dioxol-5-yl-2,2-d₂)acetamide (intermediate 19-4)

**Intermediate 19-3** (1.8 g) was dissolved in acetic acid (25 mL), acetic anhydride (1.84 g) and reduced iron powder (4.76 g) were added, and the mixture was stirred at room temperature for 1 h. After the reaction was completed, the mixture was filtered. The filtrate was concentrated to dryness under reduced pressure, and the residue was purified by column chromatography (ethyl acetate/petroleum ether = 5:1) to give the title compound (1.5 g).

MS m/z (ESI): 224.1 [M+H]⁺.

### Step 4: Synthesis of N-(6-(2-bromoacetyl)benzo[d][1,3]dioxol-5-yl-2,2-d₂)acetamide (intermediate 19-5)

A solution of HBr in acetic acid (2.39 g, 33% content) was added dropwise to a solution of **intermediate 19-4** (1.45 g) in anhydrous acetic acid (25 mL), and then Br2 (1.07 g) was added dropwise. After the dropwise addition was completed, the mixture was stirred at room temperature for 1 h. After the reaction was completed, the reaction mixture was concentrated to dryness under reduced pressure. The residue was added to water (50 mL), and the mixture was extracted with ethyl acetate (50 mL × 2). The organic phase was washed with saturated brine (50 mL), dried over anhydrous sodium sulfate, and filtered. The residue was then purified by column chromatography (ethyl acetate/petroleum ether = 5:1) to give the title compound (1.3 g).

MS m/z (ESI): 302.1 [M+H]⁺.

### Step 5: Synthesis of 1-(6-aminobenzo[d][1,3]dioxol-5-yl-2,2-d₂)-2-chloroethane-1-one (intermediate 19-6)

**Intermediate 19-5** (1.2 g) and concentrated hydrochloric acid (144.82 mg) were dissolved in ethanol (15 mL), and the reaction mixture was stirred at 60 °C for 16 h. After the reaction was completed, the reaction mixture was concentrated to dryness under reduced pressure. The residue was purified by high-performance liquid chromatography (YMC-Actus Triart C18 column, 5 µm silica, 30 mm in diameter, 150 mm in length; a mixture of water (containing 0.05% NH₄HCO₃) and acetonitrile with decreasing polarity was used as the eluent (acetonitrile gradient: 40%-50%)) to give the title compound (577 mg).

MS m/z (ESI): 216.0 [M+H]⁺.

### Step 6: Synthesis of (S)-14-(chloromethyl)-7-ethyl-7-hydroxy-10,13-dihydro-11H-[1,3]dioxolo[4,5-g]pyrano[3',4':6,7]indolizino[1,2-b]quinolin-8,11(7H)-dione-2,2-d₂ (intermediate 19-7)

**Intermediate 19-6** (100.0 mg) and **intermediate 1-3** (109.87 mg) were dissolved in toluene (1 mL) and acetic acid (1 mL), and pyridinium p-toluenesulfonate (5.24 mg) was added thereto. The reaction mixture was stirred at 100 °C for 16 h. After the reaction was completed, the reaction mixture was cooled to room temperature and then directly concentrated to dryness under reduced pressure. Ethanol (5 mL) was added, and the reaction mixture was stirred at 25 °C for 0.5 h. The reaction mixture was then filtered, and the filter cake was washed with ethanol (5 mL × 2) to give the title compound (100.0 mg).

MS m/z (ESI): 443.0 [M+H]⁺.

### Step 7: Synthesis of (S)-14-(aminomethyl)-7-ethyl-7-hydroxy-10,13-dihydro-11H-[1,3]dioxolo[4,5-g]pyrano[3',4':6,7]indolizino[1,2-b]quinolin-8,11(7H)-dione-2,2-d₂ (intermediate 19-8)

**Intermediate 19-7** (100.00 mg) was dissolved in absolute ethanol (1.5 mL) and anhydrous *N,N*-dimethylformamide (1.5 mL), and urotropin (94.97 mg) was added thereto. The reaction mixture was stirred at 50 °C for 6 h. After the reaction was completed, the reaction mixture was concentrated to dryness under reduced pressure. The residue was purified by high-performance liquid chromatography (column: Boston Green ODS 150 × 30 mm × 5 µm; mobile phase: [A: water (formic acid), B: acetonitrile]; B%: 0%-30%, 12 min) to give the title compound (25.0 mg).

MS m/z (ESI): 424.0 [M+H]⁺.

### Step 8: Synthesis of 2-cyclopropyl-N-(((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)methyl)-2-hydroxyacetamide (compound 19)

**Intermediate 19-8** (7 mg) and **intermediate 11-1** (5.76 mg) were dissolved in anhydrous *N,N-*dimethylformamide (0.5 mL), and *O*-(7-azabenzotriazol-1-yl)-*N,N,N',N'*-tetramethyluronium hexafluorophosphate (12.57 mg) and diisopropylethylamine (4.27 mg) were added thereto. The reaction mixture was stirred at 25 °C for 1 h. After the reaction was completed, the reaction mixture was filtered and purified by preparative high-performance liquid chromatography (Waters Xbridge C18 column, 5 µm, 25 mm in diameter, 100 mm in length; a mixture of water (containing 0.05% formic acid) and acetonitrile with decreasing polarity was used as the eluent (acetonitrile gradient: 20%-50%, elution time: 12 min)) to give the title compound (2.60 mg).

MS m/z (ESI): 522.1 [M+H]⁺.

**¹H NMR (400 MHz, DMSO-*d*₆)** δ = 8.62 (t, *J =* 5.9 Hz, 1H), 7.84 (s, 1H), 7.51 (s, 1H), 7.24 (s, 1H), 6.49 (s, 1H), 5.48-5.41 (m, 5H), 4.72 (d, *J =* 5.5 Hz, 2H), 3.59-3.52 (m, 1H), 2.00-1.76 (m, 2H), 1.05-0.96 (m, 1H), 0.88 (t, *J =* 7.4 Hz, 3H), 0.37-0.30 (m, 2H), 0.29-0.19 (m, 2H).

### Step 9: Synthesis of 2-cyclopropyl-N-(((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)methyl)-2-hydroxyacetamide (compound 19-P1/P2)

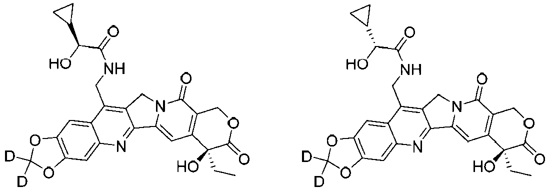

**Intermediate 19-8** (7 mg) and **intermediate 14-10-P1** (5.76 mg) were dissolved in anhydrous *N,N*-dimethylformamide (0.5 mL), and *O*-(7-azabenzotriazol-1-yl)-*N,N,N',N'*-tetramethyluronium hexafluorophosphate (12.57 mg) and diisopropylethylamine (4.27 mg) were added thereto. The reaction mixture was stirred at 25 °C for 1 h. After the reaction was completed, the reaction mixture was concentrated to dryness under reduced pressure. The residue was purified by preparative high-performance liquid chromatography (Waters Xbridge C18 column, 5 µm, 25 mm in diameter, 100 mm in length; a mixture of water (containing 0.05% formic acid) and acetonitrile with decreasing polarity was used as the eluent; acetonitrile gradient: 15%-45%, elution time: 12 min) to give **compound 19-P1** (3.30 mg).

MS m/z (ESI): 522.1 [M+H]⁺.

**¹H NMR (400 MHz, DMSO-*d*₆)** δ = 8.62 (t, *J =* 6.0 Hz, 1H), 7.86 (s, 1H), 7.52 (s, 1H), 7.25 (s, 1H), 6.51 (s, 1H), 5.54-5.51 (m, 1H), 5.47 (s, 2H), 5.43 (s, 2H), 4.72 (d, *J=* 6.0 Hz, 2H), 3.55-3.53 (m, 1H), 1.94-1.78 (m, 2H), 1.05-0.96 (m, 1H), 0.88 (t, *J =* 7.3 Hz, 3H), 0.40-0.30 (m, 2H), 0.29-0.19 (m, 2H).

**Intermediate 19-8** (7 mg) and **intermediate 14-10-P2** (5.76 mg) were dissolved in anhydrous N,N-dimethylformamide (0.5 mL), and *O*-(7-azabenzotriazol-1-yl)-*N,N,N',N'*-tetramethyluronium hexafluorophosphate (12.57 mg) and diisopropylethylamine (4.27 mg) were added thereto. The reaction mixture was stirred at 25 °C for 1 h. After the reaction was completed, the reaction mixture was concentrated to dryness under reduced pressure. The residue was purified by preparative high-performance liquid chromatography (Waters Xbridge C18 column, 5 µm, 25 mm in diameter, 100 mm in length; a mixture of water (containing 0.05% formic acid) and acetonitrile with decreasing polarity was used as the eluent (acetonitrile gradient: 15%-45%, elution time: 12 min) to give **compound 19-P2** (4.0 mg).

MS m/z (ESI): 522.1 [M+H]⁺.

**¹H NMR (400 MHz, DMSO-*d*₆)** δ = 8.62 (t, *J =* 6.1 Hz, 1H), 7.86 (s, 1H), 7.52 (s, 1H), 7.25 (s, 1H), 6.50 (s, 1H), 5.54-5.51 (m, 1H), 5.46 (s, 2H), 5.43 (s, 2H), 4.72 (d, *J=* 5.8 Hz, 2H), 3.55-3.52 (m, 1H), 1.94-1.80 (m, 2H), 1.04-0.95 (m, 1H), 0.88 (t, *J =* 7.4 Hz, 3H), 0.39-0.30 (m, 2H), 0.29-0.21 (m, 2H).

The two isomers were separately further analyzed by the following chiral supercritical fluid chromatography analysis method.

| | |
|---|---|
| Column | Chiralcel OD-3 50A 4.6mm I.D., 3µm |
| Mobile phase | A: carbon dioxide |
| | B: ethanol (0.05% diethylamine) |
| Flow rate | 4 mL/min |
| Wavelength | PDA 254nm |
| Column temperature | 35°C |
| Column pressure | 1500 Psi |
| Instrument model | Waters UPCC with PDA Detector and QDa Detector |

### Compound 19-P1:

Under the chiral high-performance liquid chromatographic conditions described above, the retention time was 2.877 min.

### Compound 19-P2:

Under the chiral high-performance liquid chromatographic conditions described above, the retention time was 2.690 min.

### Confirmation of configuration of compound 19-P1 (X-ray single-crystal diffraction method)

Single crystal culture method: 10 mg of a sample of compound 19-P1 was weighed out and placed in a 1.5 mL centrifuge tube, and 300 µL of pyridine was added. After complete dissolution by sonication, the tube was sealed with a sealing film on which three small holes were made with a needle for slow volatilization at 20-30 °C for 48 h to give a needle-like crystal.

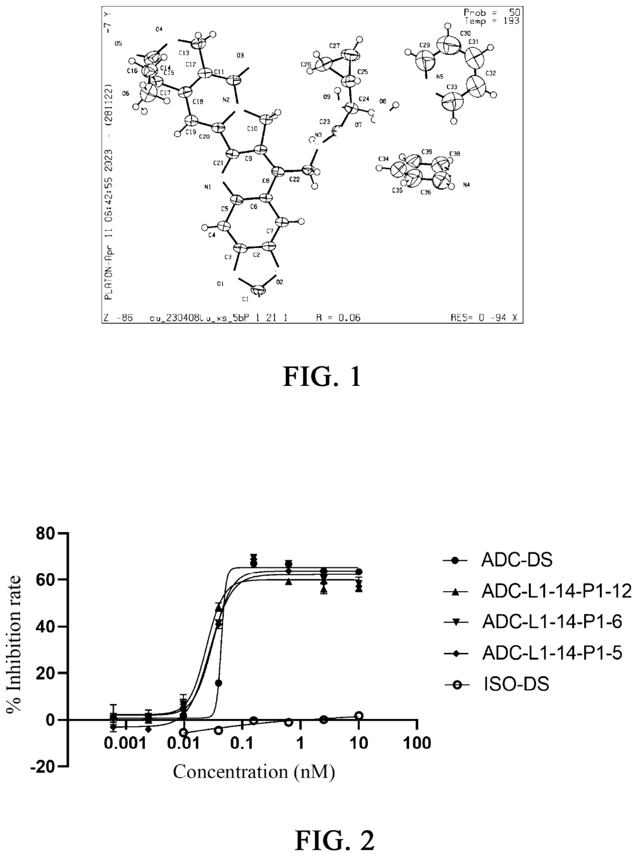

The resulting single crystal sample was subjected to X-ray analysis. The test results are shown in Table 1 and FIG. 1.

**Table 1. Single crystal sample and crystal data of compound 19-P1**

| | |
|---|---|
| Chemical formula | C₂₇ H₂₃ D₂ N₃ O₈, H₂ O, 2(C₅ H₅ N) |
| Chemical formula weight | 697.73 |
| Crystal system | Monoclinic |
| Space group | P 1 21 1 |
| a, Å | a=13.7058(5) |
| b, Å | b= 6.7026(3) |
| c, Å | c= 18.6204(7) |
| α, deg | 90 |
| β, deg | 97.727(3) |
| γ, deg | 90 |
| Volume, Å³ | 1695.02(12) |
| Z | 2 |
| D_{calc}, g cm⁻³ | 1.367 |
| Temperature, K | 193 |
| Radiation (wavelength) | Cu Kα(1.54178) |
| Instrument | BRUKER D8 VENTURE |
| Ranges of h, k, and 1 | -15<=h<=17, -8<=k<=7, -23<=1<=23 |
| Range of θ | 3.254-79.721 |
| Program | SHELXL-2014 |
| Collected data | 25136 |
| Unique data | 6674 |
| R(Fₒ) | 0.0571 |
| R_{w}(Fₒ²) | 0.1713 |
| Goodness of fit | 1.045 |
| Absolute configuration determination | 0.04(11) |

The chemical structure of **compound 19-P1** was determined by the X-ray crystal diffraction experiment described above to be:

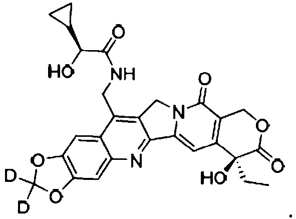

### Example 2-2: Synthesis of (S)-2-cyclopropyl-N-(((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d₂)methyl)-2-hydroxyacetamide (compound 19-S)

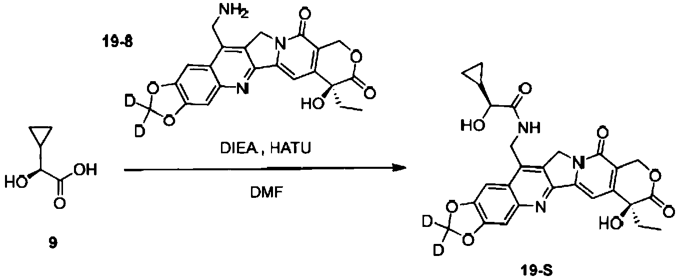

**Intermediate 19-8** (2.4 g) and **intermediate 9** (1645.5 mg) were dissolved in anhydrous *N,N-*dimethylformamide (25 mL), and *O*-(7-azabenzotriazol-1-yl)-*N,N,N,N*-tetramethyluronium hexafluorophosphate (3.24 g) and N,N-diisopropylethylamine (1465.11 mg) were added thereto. The reaction mixture was stirred at 25 °C for 3 h. After the reaction was completed, the reaction mixture was concentrated to dryness under reduced pressure. Ethyl acetate (100 mL) was added to the residue, and the mixture was stirred for 16 h and filtered. Methanol (50 mL) was then added to the filter cake, and the mixture was stirred for 16 h and filtered, thus giving the title compound (1.6 g).

MS m/z (ESI): 522.1 [M+H]⁺.

**¹H NMR (400 MHz, DMSO-*d*₆)** δ = 8.60 (t, *J =* 5.9 Hz, 1H), 7.84 (s, 1H), 7.50 (s, 1H), 7.23 (s, 1H), 6.48 (s, 1H), 5.49 (d, *J =* 5.1 Hz, 1H), 5.47-5.37 (m, 4H), 4.71 (d, *J =* 5.8 Hz, 2H), 3.54 (t, *J* = 5.6 Hz, 1H), 1.97-1.75 (m, 2H), 1.07-0.94 (m, 1H), 0.87 (t, *J =* 7.3 Hz, 3H), 0.40-0.29 (m, 2H), 0.29-0.20 (m, 2H).

**Compound 19-S** was further analyzed by the following chiral supercritical fluid chromatography analysis method.

| | |
|---|---|
| Column | Chiralcel OD-3 50A 4.6mm I.D., 3µm |
| Mobile phase | A: carbon dioxide |
| | B: ethanol (0.05% diethylamine) |
| Flow rate | 4 mL/min |
| Wavelength | PDA 254nm |
| Column temperature | 35°C |
| Column pressure | 1500 Psi |
| Instrument model | Waters UPCC with PDA Detector and QDa Detector |

Under the chiral supercritical fluid chromatographic conditions described above, the retention time of **compound 19-S** prepared in this example was 2.853 min, which was substantially consistent with the retention time (2.877 min) of **compound 19-P1** prepared in Example 2-1 under the same chromatographic analysis conditions. Thus, **compound 19-S and compound 19-P1** were determined to have the same configuration, being the same compound.

### Example 3: Synthesis of N-((12S)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl)-3,8,11,14,17-pentaoxo-5-oxa-2,7,10,13,16-pentaazaoctadecan-18-yl)-6-(2,5-dioxo-2,5-dihydro-1H-pyrrol-1-yl)hexanamide (compound L1-14) and isomers L1-14-P1 and L1-14-P2

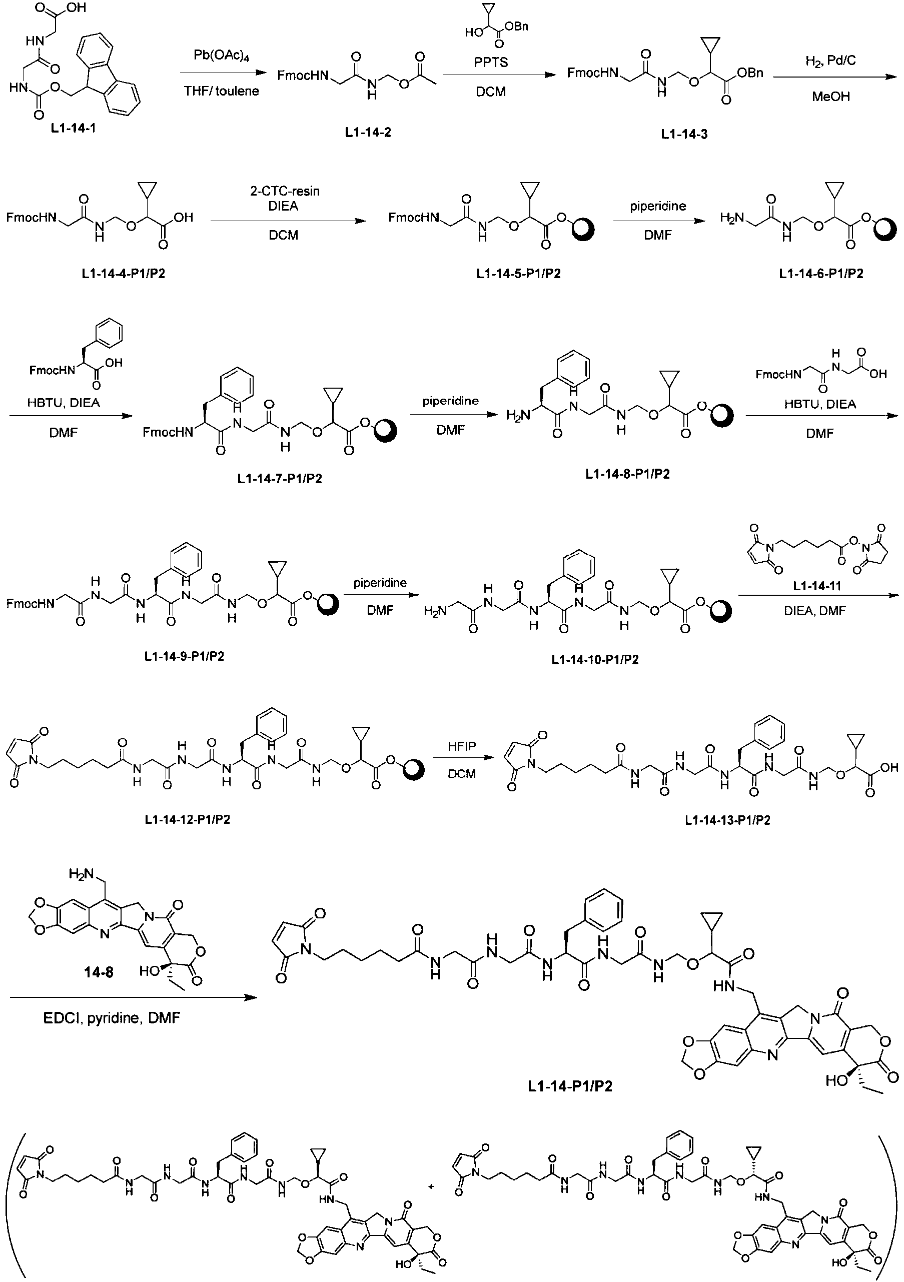

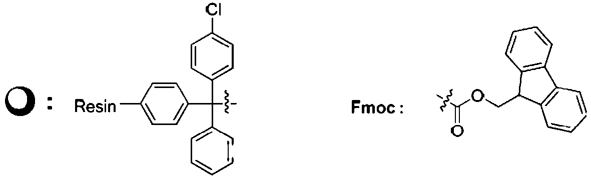

### Step 1: Synthesis of intermediate L1-14-2

**Starting material L1-14-1** (25 g), lead acetate (43.79 g), and pyridine (6.98 g) were dissolved in a mixed solvent of tetrahydrofuran (600 mL) and toluene (200 mL), and the mixture was heated to 85 °C under a nitrogen atmosphere and allowed to react with stirring for 18 h. After the reaction was completed, the reaction mixture was cooled to room temperature and filtered. The filtrate was concentrated to dryness under reduced pressure, and the residue was purified by column chromatography (chromatographic column: ISCO^{®}; 330 g SepaFlash^{®} Silica Flash Column, mobile phase gradient: 0-75% ethyl acetate/petroleum ether, flow rate: 100 mL/min) to give the title compound (18 g).

**¹H NMR (400 MHz, METHANOL-*d*₄)** δ = 7.82 (d, *J =* 7.5 Hz, 2H), 7.69 (d, *J =* 7.3 Hz, 2H), 7.44-7.38 (m, 2H), 7.36-7.31 (m, 2H), 5.22 (s, 2H), 4.39 (d, *J* = 6.8 Hz, 2H), 4.28-4.22 (m, 1H), 3.81 (s, 2H), 2.03 (s, 3H).

MS m/z (ESI): 391.1 [M+Na]⁺.

### Step 2: Synthesis of intermediate L1-14-3

**Intermediate L1-14-2** (5 g), benzyl 2-cyclopropyl-2-hydroxyacetate (8.40 g), and pyridinium *p*-toluenesulfonate (PPTS, 341.09 mg) were dissolved in dichloromethane (150 mL), and the reaction mixture was heated to 65 °C under a nitrogen atmosphere and allowed to react with stirring for 48 h. After the reaction was completed, the reaction mixture was cooled to room temperature and filtered. The filtrate was concentrated to dryness under reduced pressure, and the residue was purified by column chromatography (chromatographic column: ISCO^{®}; 120 g SepaFlash^{®} Silica Flash Column, mobile phase gradient: 0-45% ethyl acetate/petroleum ether, flow rate: 80 mL/min) and then further purified by high-performance liquid chromatography (chromatographic column: Boston Prime C18 150 × 30 mm × 5 µm; mobile phase: [A: water (0.225% formic acid), B: acetonitrile]; B%: 42%-82%, 13 min) to give the title compound (1.4 g).

MS m/z (ESI): 537.2 [M+Na]⁺.

### Step 3: Synthesis of intermediates L1-14-4-P1 and L1-14-4-P2

**Intermediate L1-14-3** (1.4 g) was dissolved in a mixed solvent of methanol (15 mL) and water (15 mL), wet palladium on carbon (10% mass content, 0.15 g) was added, and the reaction mixture was allowed to react with stirring at 25 °C for 16 h under a hydrogen atmosphere. After the reaction was completed, the reaction mixture was filtered. The filtrate was concentrated to dryness under reduced pressure, and the residue was purified by high-performance liquid chromatography (chromatographic column: Boston Prime C18 150 × 30 mm × 5 µm; mobile phase: [A: water (0.225% formic acid), B: acetonitrile]; B%: 24%-64%, 13 min) and then further purified by preparative supercritical fluid chromatography (chromatographic column: DAICEL CHIRALPAK IC column, 10 µm silica, 30 mm in diameter, 250 mm in length; isopropanol (containing 0.1% ammonia water) was used as the eluent) to give **intermediate L1-14-4-P1** (130 mg) and **intermediate L1-14-4-P2** (130 mg).

The two isomers were separately further analyzed by the following chiral high-performance liquid chromatography analysis method.

The chiral high-performance liquid chromatographic conditions were as follows:

| | |
|---|---|
| Chromatographic column | Chiralpak IC-3 100A, 4.6mm I.D., 3um |
| Mobile phase | A: carbon dioxide |
| | B: isopropanol (0.05% diethylamine) |
| Flow rate | 2.8 mL/min |
| Wavelength | PDA 220nm |
| Column temperature | 35°C |
| Column pressure | 1500 Psi |
| Instrument model | Waters UPCC with PDA Detector and QDa Detector |

### Intermediate L1-14-4-P1:

Under the chiral high-performance liquid chromatographic conditions described above, the retention time was 4.471 min.

MS m/z (ESI): 447.5 [M+Na]⁺.

### Intermediate L1-14-4-P2:

Under the chiral high-performance liquid chromatographic conditions described above, the retention time was 5.692 min.

MS m/z (ESI): 447.3 [M+Na]⁺.

### Step 4: Synthesis of intermediates L1-14-5-P1 and L1-14-5-P2

2-Chlorotrityl chloride resin (2-CTC-resin) (specification: about 1.19 mmol/g) (257 mg) was added to dichloromethane (3 mL), and then **intermediate L1-14-4-P1** (130 mg) and diisopropylethylamine (59.37 mg) were added. The reaction mixture was allowed to react with shaking on a shaker at 25 °C for 16 h under a nitrogen atmosphere. After the reaction was completed, the resin was sequentially washed with methanol (10 mL) and dichloromethane (10 mL). The procedure was repeated 3 times. After filtration, the filter cake was dried to give **intermediate L1-14-5-P1** (330 mg).

**Intermediate L1-14-5-P2** (350 mg) was prepared according to the method described above using **intermediate L1-14-4-P2** (130 mg) as the starting material.

### Step 5: Synthesis of intermediates L1-14-6-P1 and L1-14-6-P2

**Intermediate L1-14-5-P1** (330 mg) was dissolved in *N,N*-dimethylformamide (5 mL), and piperidine (1.08 g) was added. The reaction mixture was shaken on a shaker at 25 °C for 1 h. After the reaction was completed, the resin was sequentially washed with methanol (10 mL) and dichloromethane (10 mL). The procedure was repeated 3 times. After filtration, the filter cake was dried to give **L1-14-6-P1** (220 mg).

**Intermediate L1-14-6-P2** (220 mg) was prepared according to the method described above using **intermediate L1-14-5-P2** (350 mg) as the starting material.

### Step 6: Synthesis of intermediates L1-14-7-P1 and L1-14-7-P2

**Intermediate L1-14-6-P1** (220 mg) and (((9*H*-fluoren-9-yl)methoxy)carbonyl)-L-phenylalanine (230.17 mg) were dissolved in *N,N*-dimethylformamide (5 mL), and O-(benzotriazol-1-yl)-*N,N,N',N*'-tetramethyluronium hexafluorophosphate (HBTU) (225.31 mg) and diisopropylethylamine (99.96 mg) were added to the reaction mixture. The reaction mixture was shaken on a shaker at 25 °C for 1 h. After the reaction was completed, the resin was sequentially washed with methanol (10 mL) and dichloromethane (10 mL). The procedure was repeated 3 times. After filtration, the filter cake was dried to give **L1-14-7-P1** (377 mg).

**Intermediate L1-14-7-P2** (361 mg) was prepared according to the method described above using **intermediate L1-14-6-P2** (220 mg) as the starting material.

### Step 7: Synthesis of intermediates L1-14-8-P1 and L1-14-8-P2

**Intermediate L1-14-7-P1** (377 mg) was dissolved in *N,N*-dimethylformamide (5 mL), and piperidine (37.22 mg) was added. The reaction mixture was shaken on a shaker at 25 °C for 1 h. After the reaction was completed, the resin was sequentially washed with methanol (10 mL) and dichloromethane (10 mL). The procedure was repeated 3 times. After filtration, the filter cake was dried to give **L1-14-8-P1** (270 mg).

**Intermediate L1-14-8-P2** (260 mg) was prepared according to the method described above using **intermediate L1-14-7-P2** (361 mg) as the starting material.

### Step 8: Synthesis of intermediates L1-14-9-P1 and L1-14-9-P2

**Intermediate L1-14-8-P1** (270 mg) was dissolved in *N,N*-dimethylformamide (5 mL), and *N-*((9*H*-fluoren-9-ylmethoxy)carbonyl)glycylglycine (209.58 mg), O-(benzotriazol-1-yl)-*N,N,N',N*'-tetramethyluronium hexafluorophosphate (224.29 mg), and diisopropylethylamine (99.51 mg) were sequentially added. The reaction mixture was shaken on a shaker at 25 °C for 1 h. After the reaction was completed, the resin was sequentially washed with methanol (10 mL) and dichloromethane (10 mL). The procedure was repeated 3 times. After filtration, the filter cake was dried to give **intermediate L1-14-9-P1** (403 mg).

**Intermediate L1-14-9-P2** (420 mg) was prepared according to the method described above using **intermediate L1-14-8-P2** (260 mg) as the starting material.

### Step 9: Synthesis of intermediates L1-14-10-P1 and L1-14-10-P2

**Intermediate L1-14-9-P1** (403 mg) was dissolved in *N*,*N*-dimethylformamide (5 mL), and piperidine (1.08 g) was added. The reaction mixture was shaken on a shaker at 25 °C for 1 h. After the reaction was completed, the resin was sequentially washed with methanol (10 mL) and dichloromethane (10 mL). The procedure was repeated 3 times. After filtration, the filter cake was dried to give **L1-14-10-P1** (300 mg).

**Intermediate L1-14-10-P2** (320 mg) was prepared according to the method described above using **intermediate L1-14-9-P2** (420 mg) as the starting material.

### Step 10: Synthesis of intermediates L1-14-12-P1 and L1-14-12-P2

**Intermediate L1-14-10-P1** (300 mg) was dissolved in N,N-dimethylformamide (5 mL), and **compound L1-14-11** (182.13 mg) and diisopropylethylamine (99.41 mg) were sequentially added. The reaction mixture was shaken on a shaker at 25 °C for 16 h. After the reaction was completed, the resin was sequentially washed with methanol (10 mL) and dichloromethane (10 mL). The procedure was repeated 3 times. After filtration, the filter cake was dried to give **L1-14-12-P1** (374 mg).

**Intermediate L1-14-12-P2** (387 mg) was prepared according to the method described above using **intermediate L1-14-10-P2** (320 mg) and **intermediate L1-14-11** (194.27 mg) as the starting materials.

### Step 11: Synthesis of intermediates L1-14-13-P1 and L1-14-13-P2

**Intermediate L1-14-12-P1** (374 mg) was added to a mixed solvent of dichloromethane (8 mL) and hexafluoroisopropanol (HFIP, 2 mL), and the reaction mixture was allowed to react with shaking on a shaker at 25 °C for 0.5 h. After the reaction was completed, the reaction mixture was filtered to remove the resin. The filtrate was concentrated to dryness under reduced pressure, and the residue was purified by high-performance liquid chromatography (chromatographic column: Boston Prime C18 150 × 30 mm × 5 µm; mobile phase: [A: water (0.225% formic acid), B: acetonitrile]; B%: 15%-35%, 9 min) to give **intermediate L1-14-13-P1** (74 mg).

**¹H NMR (400 MHz, METHANOL-*d*₄)** δ = 7.33-7.20 (m, 5H), 6.81 (s, 2H), 4.77-4.70 (m, 2H), 4.60-4.52 (m, 1H), 3.96-3.70 (m, 6H), 3.61 (d, *J* = 7.6 Hz, 1H), 3.55-3.47 (m, 2H), 3.27-3.23 (m, 1H), 3.05-2.98 (m, 1H), 2.30 (t, *J* = 7.4 Hz, 2H), 1.72-1.55 (m, 4H), 1.38-1.29 (m, 2H), 1.16-1.07 (m, 1H), 0.60-0.47 (m, 4H).

MS m/z (ESI): 679.7 [M+Na]⁺.

**Intermediate L1-14-13-P2** (86 mg) was prepared according to the method described above using **intermediate L1-14-12-P2** (387 mg) as the starting material.

**¹H NMR (400 MHz, METHANOL-*d*₄)** δ = 7.40-7.21 (m, 5H), 6.82 (s, 2H), 4.81-4.67 (m, 2H), 4.60-4.50 (m, 1H), 3.97-3.70 (m, 6H), 3.66-3.57 (m, 1H), 3.56-3.47 (m, 2H), 3.27-3.22 (m, 1H), 3.08-2.97 (m, 1H), 2.35-2.26 (m, 2H), 1.75-1.55 (m, 4H), 1.41-1.32 (m, 2H), 1.16-1.06 (m, 1H), 0.60-0.45 (m, 4H).

MS m/z (ESI): 679.5 [M+Na]⁺.

### Step 12: Synthesis of compounds L1-14-P1 and L1-14-P2

**Intermediate L1-14-13-P1** (31.17 mg), **intermediate 14-8** (20 mg), 1-(3-dimethylaminopropyl)-3-ethylcarbodiimide hydrochloride (EDCI) (18.20 mg), pyridine (11.26 mg), and 1-hydroxybenzotriazole (12.83 mg) were dissolved in N,N-dimethylformamide (1 mL), and the reaction mixture was allowed to react with stirring at 25 °C for 2 h under a nitrogen atmosphere. After the reaction was completed, the reaction mixture was purified by high-performance liquid chromatography (chromatographic column: Boston Green ODS 150 × 30 mm × 5 µm; mobile phase:[A: water (0.225% formic acid), B: acetonitrile]; B%: 26%-46%, 12 min) to give **compound L1-14-P1** (12.3 mg).

**¹H NMR (400 MHz, DMSO-*d*₆)** δ = 8.65 *(t, J* = 5.9 Hz, 1H), 8.58 (t, *J =* 6.6 Hz, 1H), 8.27 (t, *J* = 5.6 Hz, 1H), 8.17-8.02 (m, 2H), 7.99 (t, *J =* 5.6 Hz, 1H), 7.79 (s, 1H), 7.52 (s, 1H), 7.28-7.19 (m, 5H), 7.19-7.14 (m, 1H), 6.98 (s, 2H), 6.49 (s, 1H), 6.29 (d, *J =* 3.7 Hz, 2H), 5.48-5.37 (m, 4H), 4.85-4.70 (m, 2H), 4.70-4.65 (m, 1H), 4.52-4.45 (m, 1H), 4.43-4.37 (m, 1H), 3.79-3.54 (m, 7H), 3.49 (d, *J=* 7.1 Hz, 1H), 3.08-3.01 (m, 1H), 2.85-2.74 (m, 1H), 2.14-2.06 (m, 2H), 1.94-1.80 (m, 2H), 1.52-1.42 (m, 4H), 1.27-1.13 (m, 2H), 1.01-0.92 (m, 1H), 0.88 (t, *J =* 7.3 Hz, 3H), 0.41-0.28 (m, 4H).

MS m/z (ESI): 1060.3 [M+H]⁺.

**Compound L1-14-P2** (11.4 mg) was prepared according to the method described above using **intermediate L1-14-12-P2** (31.17 mg) and **intermediate 14-8** (20 mg) as the starting materials.

**¹H NMR (400 MHz, DMSO-*d*₆)** δ = 8.72-8.52 (m, 2H), 8.33-8.25 (m, 1H), 8.17-7.94 (m, 3H), 7.80 (s, 1H), 7.51 (s, 1H), 7.32-7.19 (m, 5H), 7.18-7.12 (m, 1H), 6.99 (s, 2H), 6.49 (s, 1H), 6.35-6.25 (m, 2H), 5.48-5.36 (m, 4H), 4.84-4.59 (m, 3H), 4.54-4.45 (m, 1H), 4.44-4.35 (m, 1H), 3.81-3.54 (m, 7H), 3.49 (d, *J=* 6.7 Hz, 1H), 3.08-3.01 (m, 1H), 2.87-2.73 (m, 1H), 2.14-2.05 (m, 2H), 1.95-1.77 (m, 2H), 1.53-1.39 (m, 4H), 1.25-1.13 (m, 2H), 1.03-0.82 (m, 4H), 0.43-0.25 (m, 4H)

MS m/z (ESI): 1060.3 [M+H]⁺.

The two isomers were separately further analyzed by the following chiral high-performance liquid chromatography method.

The chiral high-performance liquid chromatographic conditions were as follows:

| | |
|---|---|
| Chromatographic column | Chiralcel OJ-3 100A, 4.6mm I.D., 3um |
| Mobile phase | A: carbon dioxide |
| | B: ethanol (0.05% diethylamine) |
| Flow rate | 2.8 mL/min |
| Wavelength | PDA 220nm |
| Column temperature | 35°C |
| Column pressure | 1500 Psi |
| Instrument model | Waters UPCC with PDA Detector and QDa Detector |

Under the chiral high-performance liquid chromatographic conditions described above, the retention time of **compound L1-14-P1** was 3.735 min.

Under the chiral high-performance liquid chromatographic conditions described above, the retention time of **compound L1-14-P2** was 3.901 min.

### Example 4-1: Synthesis of N-((125)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d2)-3,8,11,14,17-pentaoxo-5-oxa-2,7,10,13,16-pentaazaoctadecan-18-yl)-6-(2,5-dioxo-2,5-dihydro-1H-pyrrol-1-yl)hexanamide (compound L1-19-P1)

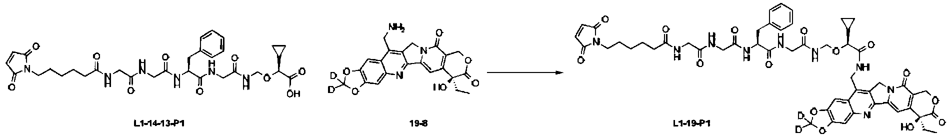

**Intermediate L1-14-13-P1** (7.75 mg), **intermediate 19-8** (5.0 mg), 1-(3-dimethylaminopropyl)-3-ethylcarbodiimide hydrochloride (4.53 mg), pyridine (2.80 mg), and 1-hydroxybenzotriazole (3.19 mg) were dissolved in *N,N*-dimethylformamide (1 mL), and the reaction mixture was allowed to react with stirring at 25 °C for 2 h under a nitrogen atmosphere. After the reaction was completed, the reaction mixture was purified by high-performance liquid chromatography (column: Boston Green ODS 150 × 30 mm × 5 µm; mobile phase: [A: water (0.225% formic acid), B: acetonitrile]; B%: 22%-52%, 12 min) to give **L1-19-P1** (6.0 mg).

MS m/z (ESI): 1062.3 [M+H]⁺.

**¹H NMR (400 MHz, DMSO-*d*₆)** δ = 8.74-8.69 (m, 1H), 8.59 (t, *J=* 6.7 Hz, 1H), 8.32-8.26 (t, *J =* 5.6 Hz, 1H), 8.11 (d, *J =* 7.8 Hz, 1H), 8.06 *(t, J=* 5.3 Hz, 1H), 7.99-7.94 (m, 1H), 7.80 (s, 1H), 7.52 (s, 1H), 7.27-7.20 (m, 5H), 7.19-7.11 (m, 1H), 6.99 (s, 2H), 6.50 (s, 1H), 5.49-5.41 (m, 4H), 4.84-4.72 (m, 2H), 4.71-4.63 (m, 1H), 4.53-4.48 (m, 1H), 4.44-4.36 (m, 1H), 3.77-3.56 (m, 6H), 3.49 (d, *J=* 7.0 Hz, 1H), 3.06-3.02 (m, 1H), 2.83-2.74 (m, 1H), 2.10 (t, *J =* 7.6 Hz, 2H), 1.91-1.82 (m, 2H), 1.50-1.42 (m, 4H), 1.22-1.13 (m, 2H), 1.02-0.90 (s, 1H), 0.88 (t, *J=* 7.3 Hz, 3H), 0.38-0.29 (m, 4H).

**L1-19-P1** was further analyzed by the following chiral high-performance liquid chromatography analysis method.

The chiral high-performance liquid chromatographic conditions were as follows:

| | |
|---|---|
| Chromatographic column | Chiralcel OJ-3 100A, 4.6mm I.D., 3um |
| Mobile phase | A: carbon dioxide |
| | B: ethanol (0.05% diethylamine) |
| Flow rate | 2.8 mL/min |
| Wavelength | PDA 220nm |
| Column temperature | 35°C |
| Column pressure | 1500 Psi |
| Instrument model | Waters UPCC with PDA Detector and QDa Detector |

Under the chiral supercritical fluid chromatographic conditions described above, the retention time of **L1-19-P1** was 3.775 min.

### Example 4-2: N-((4S,12S)-12-benzyl-4-cyclopropyl-1-((S)-7-ethyl-7-hydroxy-8,11-dioxo-7,8,11,13-tetrahydro-10H-[1,3]dioxolo[4,5-g]pyrano[3',4':6,7]indolizino[1,2-b]quinolin-14-yl-2,2-d2)-3,8,11,14,17-pentaoxo-5-oxa-2,7,10,13,16-pentaazaoctadecan-18-yl)-6-(2,5-dioxo-2,5-dihydro-1H-pyrrol-1-yl)hexanamide (compound L1-19-S)

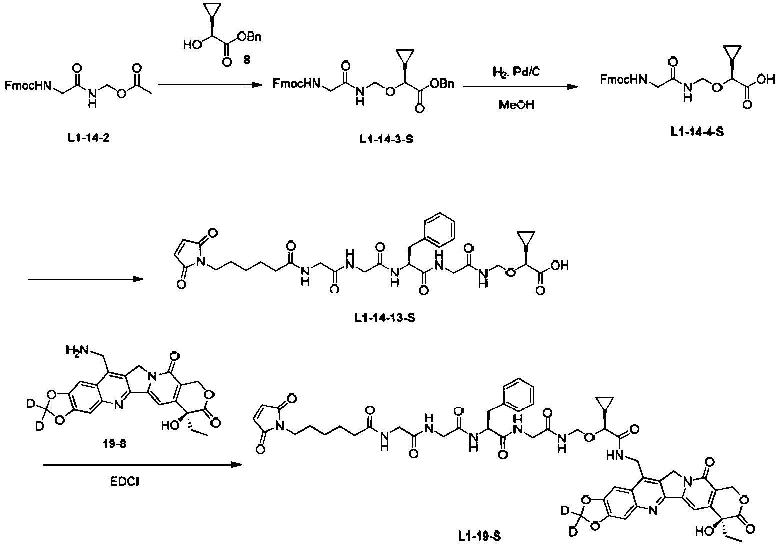

### Step 1: Synthesis of L1-14-3-S

**Intermediate L1-14-2** (28 g) and **intermediate 8** (23.5 g, prepared in Example 1-2) were dissolved in dichloromethane (25 mL), silver trifluoromethanesulfonate (139.50 mg) was added to the reaction mixture, and the reaction mixture was allowed to react with stirring at 25 °C for 108 h under a nitrogen atmosphere. After the reaction was completed, the reaction mixture was filtered. The filtrate was concentrated to dryness under reduced pressure, and the residue was purified by a flash silica gel column (mobile phase gradient: tetrahydrofuran/dichloromethanol: 0-7%, flow rate: 70 mL/min) to give the title compound (10.3 g).

MS m/z (ESI): 537.3 [M+Na]⁺.

### Step 2: Synthesis of L1-14-4-S

**Intermediate L1-14-3-S** (10 g) was dissolved in tetrahydrofuran (100 mL), wet palladium on carbon (10% mass content, 1 g) was added, and the reaction mixture was allowed to react with stirring at 0 °C for 16 h under a hydrogen atmosphere. After the reaction was completed, the reaction mixture was filtered. The filtrate was concentrated to dryness under reduced pressure, and petroleum ether/ethyl acetate (10 mL/0.5 mL) was added to the residue. The mixture was stirred for 2 h and filtered to give the title compound (6 g).

MS m/z (ESI): 447.2 [M+Na]⁺.

**L1-14-4-S** was further analyzed by the following chiral high-performance liquid chromatography analysis method.

The chiral high-performance liquid chromatographic conditions were as follows:

| | |
|---|---|
| Chromatographic column | Chiralpak IC-3 100A, 4.6mm I.D., 3um |
| Mobile phase | A: carbon dioxide |
| | B: isopropanol (0.05% diethylamine) |
| Flow rate | 2.8 mL/min |
| Wavelength | PDA 220nm |
| Column temperature | 35°C |
| Column pressure | 1500 Psi |
| Instrument model | Waters UPCC with PDA Detector and QDa Detector |

Under the chiral supercritical fluid chromatographic conditions described above, the retention time of **L1-14-4-S** was 4.385 min, which was substantially consistent with the retention time (4.471 min) of **compound L1-14-4-P1** under the same chromatographic analysis conditions. Thus, **L1-14-4-S** and **L1-14-4-P1** have the same configuration, being the same compound.

### Step 3: Synthesis of L1-19-S

**Intermediate L1-14-13-S** (600 mg, prepared with reference to the synthetic method for **L1-14-13-P1** using **L1-14-4-S** as the starting material), **intermediate 19-8** (502.93 mg), 1-(3-dimethylaminopropyl)-3-ethylcarbodiimide hydrochloride (EDCI) (350.31 mg), pyridine (216.82 mg), and 1-hydroxybenzotriazole (246.91 mg) were dissolved in N,Ndimethylformamide (2 mL), and the reaction mixture was allowed to react with stirring at 25 °C for 2 h under a nitrogen atmosphere. After the reaction was completed, the reaction mixture was purified by high-performance liquid chromatography (column: Boston Green ODS 150 × 30 mm × 5 µm; mobile phase: [A: water (0.05% formic acid), B: acetonitrile]; B%: 24%-54%, 12 min) to give the title compound (286 mg).

**¹H NMR (400 MHz, DMSO-*d₆*)** δ = 8.72-8.63 (m, 1H), 8.62-8.52 (m, 1H), 8.34-8.26 (m, 1H), 8.16-7.94 (m, 3H), 7.78 (s, 1H), 7.51 (s, 1H), 7.26-7.13(m, 6H), 6.99 (s, 1H), 6.56-6.42 (m, 1H), 5.52-5.40 (m, 4H), 4.85-4.60 (m, 3H), 4.59-4.46 (m, 1H), 4.44-4.38 (m, 1H), 3.79-3.53 (m, 6H), 3.52-3.44 (m, 2H), 3.08-3.02 (m, 1H), 2.85-2.75 (m, 1H), 2.09 (t, J=7.8 Hz, 2H), 1.95-1.75 (m, 2H), 1.58-1.38 (m, 4H), 1.26-1.09 (m, 2H), 1.04-0.96 (m, 1H), 0.88 (t, *J=* 7.1 Hz, 3H), 0.42-0.26 (m, 4H).

MS m/z (ESI): 1062.5 [M+H]⁺.

**L1-19-S** was further analyzed by the following chiral high-performance liquid chromatography method.

The chiral high-performance liquid chromatographic conditions were as follows:

| | |
|---|---|
| Chromatographic column | Chiralcel OJ-3 100A, 4.6mm I.D., 3um |
| Mobile phase | A: carbon dioxide |
| | B: ethanol (0.05% diethylamine) |
| Flow rate | 2.8 mL/min |
| Wavelength | PDA 220nm |
| Column temperature | 35°C |
| Column pressure | 1500 Psi |
| Instrument model | Waters UPCC with PDA Detector and QDa Detector |

Under the chiral supercritical fluid chromatographic conditions described above, the retention time of **L1-19-S** was 3.748 min, which was substantially consistent with the retention time (3.775 min) of **compound L1-19-P1** prepared in Example 4-1 under the same chromatographic analysis conditions. Thus, **L1-19-S** and **L1-19-P1** were determined to have the same configuration, belonging to the same compound.

### Example 5: Construction and production of anti-human CDH6 antibodies

The heavy and light chain variable region sequences of the anti-human CDH6 monoclonal antibodies (CDH6-Ab-4, CDH6-Ab-3, CDH6-Ab-2, and CDH6-Ab) are shown in Table 2 below, and the CDR sequences of the antibodies defined according to the Kabat numbering scheme are shown in Table 3 below. The nucleic acid sequences encoding VHs and VLs of the antibodies were recombined into expression vector pTT5 carrying a signal peptide (MGWSWILLFLLSVTAGVHS, SEQ ID NO: 29) and heavy chain constant region/light chain constant region sequences to give recombinant plasmids for VH-CH/VL-CL expression. The plasmid and the transfection reagent PEI (Polysciences, Cat. No. 24765-1) were added to OPTI-MEM (Gibco, Cat. No. 11058021). The mixture was well mixed and left to stand for 15 min. Expi293F cells (Thermofisher, Cat. No. A14527) were added, and the system was incubated on a shaker at 37 °C with 5% CO₂ at 120 rpm. On day 2 of the transfection, OPM-293 ProFeed (Shanghai OPM Biosciences Co., Ltd., Cat. No. F081918-001) and 6 g/L glucose (Sigma, Cat. No. G7528) were added. On day 6 of the transfection, the cell supernatant was collected. The sample was purified by Protein A (GE, Cat. No. 28985254), and the eluted sample was dialyzed against PBS (pH 7.4) to give an anti-human CDH6 monoclonal antibody. CDH6-Ab served as a positive control human CDH6 antibody, with its sequence derived from patent WO2018212136A1.

**Table 2. Heavy and light chain variable region sequences of anti-human CDH6 monoclonal antibodies**

| Antibody name | Sequence No. | Sequence information |
|---|---|---|
| CDH6-Ab-4-VH | SEQ ID NO:1 | EVQLVQSGAEVKKPGSSVKVSCKASGYTFTNYWMH WVRQAPGQGLEWMGDIDPSHSETHYTQKFKDRVTI TVDKSTSTAYMELSSLRSEDTAVYYCARGDLGRFDY WGQGTTVTVSS |
| CDH6-Ab-4-VL | SEQ ID NO:2 | DIQMTQSPSSLSASVGDRVTITCRASESVSIHGTHLM HWYQQKPGKPPKLLIYAASNLESGVPSRFSGSGSETD FTFTISSLQPEDIATYYCQQSFEDPWTFGGGTKVEIK |
| CDH6-Ab-3-VH | SEQ ID NO:3 | EVQLVQSGAEVKKPGASVKVSCKASGYTFTDYYMA WVRQAPGQGLEWMGYIFPNSGGTDYSQKFKGRVTL TVDKSTSTVYMELSSLRSEDTAVYYCARELGADYG DYYAMDHWGQGTTVTVSS |
| CDH6-Ab-3-VL | SEQ ID NO:4 | DIQMTQSPSSLSASVGDRVTITCRASENIDSYLAWYQ QKPGKSPKLLVYSATLLADGVPSRFSGSGSGTDYTLT ISSLQPEDFATYYCQHYYRTPPTFGQGTKLEIK |
| CDH6-Ab-2-VH | SEQ ID NO:5 | EVQLVQSGAEVKKPGASVKVSCKASGYTFTDNYIN WVRQAPGQRLEWMGWIYPGTGNSYYNEKFKGRVTI TVDTSASTAYMELSSLRSEDTAVYLCASSYGDSFDY WGQGTTVTVSS |
| CDH6-Ab-2-VL | SEQ ID NO:6 | DIVMTQSPDSLAVSLGERATINCRASQDIRNYLNWY QQKPGQTPKLLISYTSRLHSGVPDRFSGSGSGTDFTL TISSLQAEDVAVYYCQQYSQLPWTFGGGTKVEIK |
| CDH6-Ab-VH | SEQ ID NO:7 | EVQLVQSGAEVKKPGASVKVSCKASGYTFTRNFMH WVRQAPGQGLEWMGWIYPGDGETEYAQKFQGRVT ITADTSTSTAYMELSSLRSEDTAVYYCARGVYGGFA GGYFDFWGQGTLVTVSS |
| CDH6-Ab-VL | SEQ ID NO:8 | DIQMTQSPSSLSASVGDRVTITCKASQNIYKNLAWY QQKPGKAPKLLIYDANTLQTGVPSRFSGSGSGSDFTL TISSLQPEDFATYFCQQYYSGWAFGQGTKVEIK |
| human CH | SEQ ID NO:9 | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCP PCPAPELLGGPSVFLFPPKPKDTLMISRTPEVTCVVV DVSHEDPEVKFNWYVDGVEVHNAKTKPREEQYNST YRVVSVLTVLHQDWLNGKEYKCKVSNKALPAPIEK TISKAKGQPREPQVYTLPPSREEMTKNQVSLTCLVK GFYPSDIAVEWESNGQPENNYKTTPPVLDSDGSFFLY SKLTVDKSRWQQGNVFSCSVMHEALHNHYTQKSLS LSPGK |
| human CL | SEQ ID NO:10 | |

**Table 3. CDR sequences of anti-human CDH6 monoclonal antibodies (defined according to the Kabat numbering scheme)**

| Antibody name | CDR1 | Sequence No. | CDR2 | Sequence No. | CDR3 | Sequence No. |
|---|---|---|---|---|---|---|
| CDH6-Ab-4-VH | NYWMH | SEQ ID NO: 11 | DIDPSHS ETHYTQ KFKD | SEQ ID NO: 12 | GDLGRF DY | SEQ ID NO: 13 |
| CDH6-Ab-4-VL | RASESV SIHGTH LMH | SEQ ID NO: 14 | AASNLE S | SEQ ID NO: 15 | QQSFED PWT | SEQ ID NO: 16 |
| CDH6-Ab-3-VH | DYYMA | SEQ ID NO: 17 | YIFPNSG GTDYSQ KFKG | SEQ ID NO: 18 | ELGADY GDYYA MDH | SEQ ID NO: 19 |
| CDH6-Ab-3-VL | RASENI DSYLA | SEQ ID NO: 20 | SATLLA D | SEQ ID NO: 21 | QHYYR TPPT | SEQ ID NO: 22 |
| CDH6-Ab-2-VH | DNYIN | SEQ ID NO: 23 | WIYPGT GNSYYN EKFKG | SEQ ID NO: 24 | SYGDSF DY | SEQ ID NO: 25 |
| CDH6-Ab-2-VL | RASQDI RNYLN | SEQ ID NO: 26 | YTSRLH S | SEQ ID NO: 27 | QQYSQL PWT | SEQ ID NO: 28 |

### Example 6: Preparation method for antibody-drug conjugates

Conjugation: The antibody prepared in Example 5 was buffer-exchanged into a solution containing 20 mM PB, 150 mM NaCl, and 1 mM EDTA (pH 6.5) by dialysis. An 8-fold amount of a 10 mM tris(2-carboxyethyl)phosphine solution (TCEP, Thermo Scientific#77720) was added to the antibody solution, and the mixed solution was incubated in a constant-temperature metal shaker at 37 °C for 2.5 h to reduce the antibody. A 15-fold amount of the linker-payload compound (prepared in Examples 3 and 4) was dissolved in DMSO, and the resulting solution was added to the reaction system. The reaction mixture was incubated at 25 °C for 6 h for conjugation. The reaction product was desalted and buffer-exchanged into phosphate-buffered saline (PBS) through a G25 column to remove free unreacted small-molecule toxins. The ADC product was analyzed for purity and DAR value using SEC and LC-MS methods.

SEC purity analysis: The test protein sample was analyzed using the SEC-HPLC method to characterize the molecular size homogeneity of the recombinant protein and determine the purity of the recombinant protein. In this method, the HPLC instrument used was Agilent 1260, the chromatographic column was TSKgel G3000SWXL (purchased from Tosoh Bioscience), the mobile phase was 200 mM phosphate buffer, pH 7.0/isopropanol (Merck, 1.01040.4008) (v/v 9:1), the detection temperature was 25 °C, the flow rate was 0.5 mL/min, the detection wavelength was 280 nm, the loading amount of the target protein was 50 µg, and the analysis time was 40 min.

DAR value determination: The DAR value of the ADC molecule was determined using the ultra-high-performance liquid chromatography-mass spectrometry (UHPLC-MS) method. Firstly, the test ADC molecule was treated with PNGase F (NEB #P0705L) to remove N glycosylation modification, then treated with dithiothreitol (DTT, Sigma #646563), incubated at 37 °C for 1 h for reduction into light and heavy chains, and then analyzed using a Thermo Vanquish UHPLC-Q Exactive Plus mass spectrometry system. Subsequently, 2 µg of the protein was injected into a Waters ACQUITY Protein BEH molecular exclusion chromatographic column, the mobile phase was an aqueous solution containing 0.1% formic acid, 0.05% TFA, and 25% acetonitrile, the flow rate was 0.2 mL/min, the analysis time was 30 min, the mass spectrometer was Thermo Q Exactive Plus, and the main parameters of the mass spectrometry were a spray voltage of 3.8 kV, a capillary heating temperature of 300 °C, a sheath gas flow rate of 35 arb, a precursor ion scanning range of 800-3000, etc. Finally, deconvolution processing was performed through the Respect algorithm using the mass spectrum data analysis software Biopharma Finder 4.1, and the molecular weight information of mass spectrum peaks of the light and heavy chains and the mass spectrum response signals of each component were separately calculated so as to calculate the DAR value of the test ADC sample.

**Table 4. DAR values and SEC purity of CDH6-ADCs**

| ADC No. | ADC structure | DAR | SEC purity (monomer %) |
|---|---|---|---|
| ADC-L1-14-P1-12 | 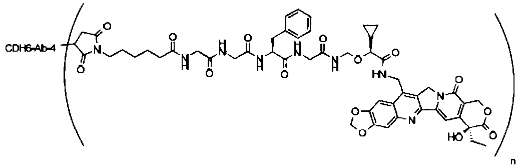 | 7.2 | 94.54 |
| ADC-L1-19-P1-12 | 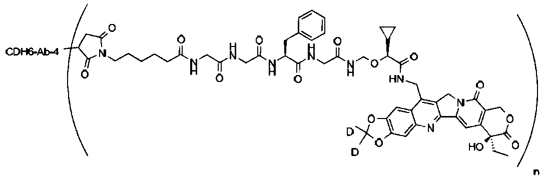 | 7.8 | 94.59 |
| ADC-L1-14-P1-6 | 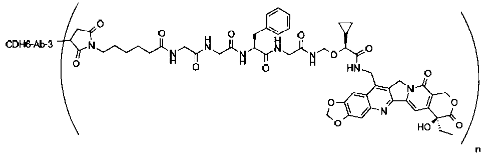 | 7.6 | 92.89 |
| ADC-L1-19-P1-6 | 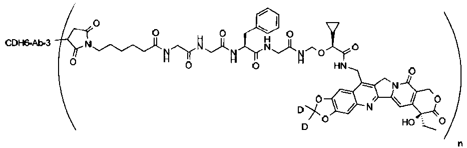 | 8.0 | 92.86 |
| ADC-L1-14-P1-5 | 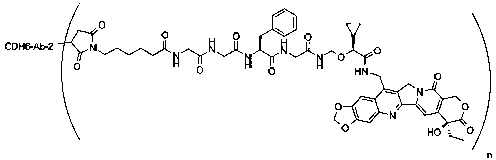 | 7.8 | 97.05 |
| ADC-L1-19-P1-5 | 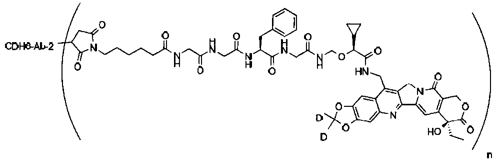 | 8.2 | 96.03 |
| ADC-DS | 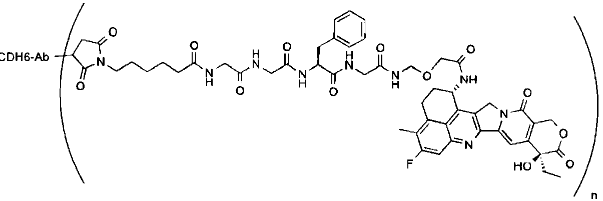 | 7.5 | 92.83 |

Using the same method as described above, the isotype control (ISO), the anti-FITC-hIgG1 antibody, was prepared and conjugated with the linker-payload compound to give an isotype control ADC. The DAR values and SEC results of the ADCs are shown below.

| ADC No. | ADC structure | DAR | SEC purity (monomer %) |
|---|---|---|---|
| ISO-DS | 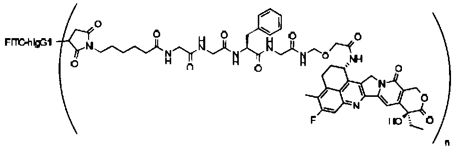 | 7.8 | 94.28 |
| ISO-L1-14-P1 | 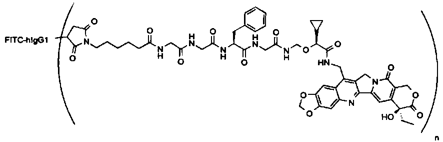 | 7.8 | 92.96 |
| ISO-L1-19-P1 | 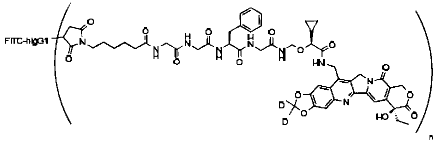 | 7.6 | 90.03 |

### Example 7. Assay for inhibition of in vitro proliferation of tumor cells by ADCs

**Cells and materials:** The human ovarian cancer cell line OVCAR3 (a cell strain with high CDH6 expression) was purchased from ATCC (#HTB-161). The human ovarian teratoma cell line PA-1 (a cell strain with medium CDH6 expression) was purchased from Nanjing Cobioer Biosciences Co., Ltd. (#CBP60800). Bovine serum, 1640 medium, MEM medium (Gibco#11095-080), MEM NEAA (Gibco#11140-050), sodium pyruvate (Gibco#11360-070), penicillin-streptomycin, and 0.25% Trypsin-EDTA were purchased from Gibco. Bovine insulin was purchased from Solarbio. 96-well plates were purchased from Cornin. Cell-Titer Glo reagent was purchased from Promega.

**Cell culture:** The OVCAR3 cells were cultured with a 1640 medium containing 20% fetal bovine serum + 2 µg/mL bovine insulin + 1% penicillin-streptomycin at 37 °C with 5% CO₂.The PA-1 cells were cultured with an MEM medium containing 10% fetal bovine serum + 1% MEM NEAA + 1% sodium pyruvate + 1% penicillin-streptomycin at 37 °C with 5% CO₂. Cells at logarithmic growth phase were available for the experiment.

**Cell proliferation activity assay:** The Cell-Titer Glo reagent was used to assay the inhibitory activity of ADCs against the proliferation of both OVCAR3 and PA-1 cell strains. The OVCAR3 cells (5000 cells per well) and PA-1 cells (800 cells per well) were seeded in 96-well plates and cultured at 37 °C with 5% CO₂ for 24 h. Each ADC molecule was diluted with the medium for the corresponding cells described above to achieve an ADC concentration of 100 nM. Then, 3-fold gradient dilution was further performed with the medium to give a total of 8 concentrations, and 10 µL of each prepared ADC solution was transferred to the 96-well plate, yielding final concentrations ranging from 0 to 10 nM. After the test ADC solution was added, the plate was incubated at 37 °C with 5% CO₂. The OVCAR3 cells and the PA-1 cells were cultured for 5 days. The Cell-Titer Glo reagent was added and the cell activity was measured.

**Data analysis:** % inhibition (inhibition rate) was calculated and fitted to determine IC₅₀. % inhibition = 1 - 100% × (Signal - Bottom) / (Top - Bottom). Signal refers to the signal value of the ADC sample group, Bottom refers to the signal value obtained without cells but with only an equivalent volume of medium added, and Top refers to the signal value obtained without the ADC sample but with only cells added.

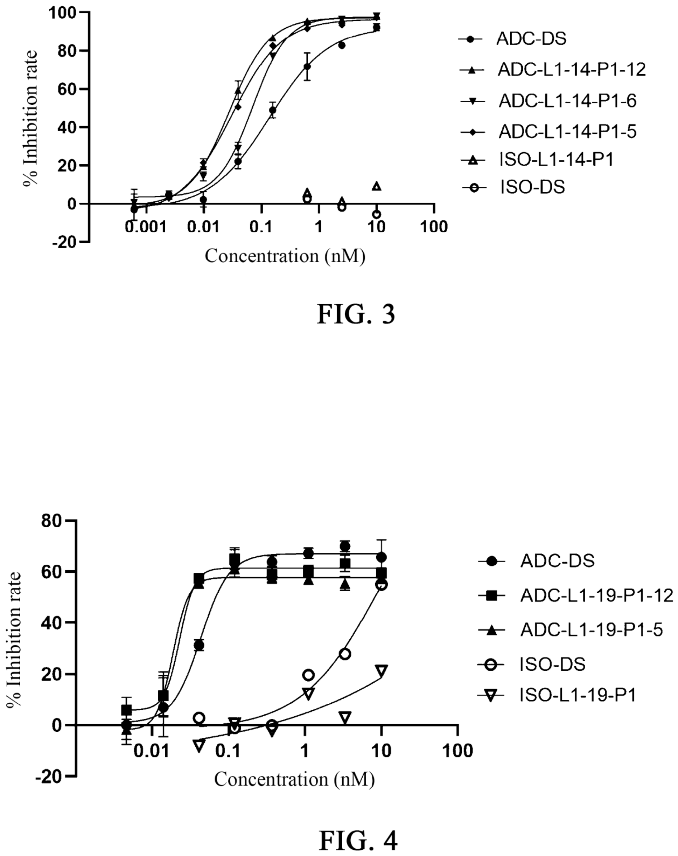

**Experimental results:** Under the conditions in this experiment, the test ADCs all exhibited strong inhibitory activity against the proliferation of the OVCAR3 and PA-1 cells, as detailed in Tables 5-6 and FIGs. 2-5.

**Table 5. Inhibitory effects of ADCs on proliferation of OVCAR3 and PA-1 cells**

| ADC No. | OVCAR3 | | PA-1 | |
|---|---|---|---|---|
| | IC₅₀ (nM) | Maximum inhibition rate (%) | IC₅₀ (nM) | Maximum inhibition rate (%) |
| ADC-L1-14-P1-12 | 0.024 | 68.2 | 0.027 | 98.2 |
| ADC-L1-14-P1-6 | 0.031 | 69.5 | 0.070 | 98.0 |
| ADC-L1-14-P1-5 | 0.028 | 68.9 | 0.030 | 97.4 |
| ADC-DS | 0.044 | 67.5 | 0.137 | 92.3 |
| ISO-DS | / | 1.9 | / | 2.7 |
| ISO-L1-14-P1 | Not determined | Not determined | / | 9.2 |

| | | | | |
|---|---|---|---|---|
| Note: "/" indicates that IC₅₀ cannot be determined by fitting. | | | | |

**Table 6. Inhibitory effects of ADCs on proliferation of OVCAR3 and PA-1 cells**

| ADC No. | OVCAR3 | | PA-1 | |
|---|---|---|---|---|
| | IC₅₀ (nM) | Maximum inhibition rate (%) | IC₅₀ (nM) | Maximum inhibition rate (%) |
| ADC-L1-19-P1-12 | 0.023 | 61.4 | 0.016 | 97.6 |
| ADC-L1-19-P1-5 | 0.019 | 57.8 | 0.018 | 96.7 |
| ADC-DS | 0.043 | 67.2 | 0.046 | 96.2 |
| ISO-DS | / | 55.1 | / | 69.5 |
| ISO-L1-19-P1 | / | 21.2 | / | 27.7 |

| | | | | |
|---|---|---|---|---|
| Note: "/" indicates that IC₅₀ cannot be determined by fitting. | | | | |

### Example 8: Evaluation of efficacy in OVCAR3 subcutaneous tumor model

**Experimental reagents:** Human ovarian cancer OVCAR3 cells were purchased from ATCC. RPMI-1640 medium was purchased from Gibco (Cat. No. A104910). Fetal bovine serum was purchased from Excell (Cat. No. FND500). Penicillin-streptomycin was purchased from Gibco (Cat. No. 15140122). Bovine insulin was purchased from Yeasen (Cat. No. 40107ES60). 0.25% trypsin-EDTA was purchased from Gibco (Cat. No. 25200-072. D-PBS (phosphate-buffered saline without calcium and magnesium ions) was purchased from Hyclone (Cat. No. SH30256.01). Matrigel was purchased from Corning (Cat. No. 356237).

### Experimental method:

Animal information: Balb/c nude mice (female, 5-6 weeks old, weighing about 14-20 g) were purchased from Beijing Vital River Biotechnology Co., Ltd. The mice were bred in an SPF-level environment within independently ventilated cages. All animals had *ad libitum* access to standard certified commercial laboratory diet and drinking water.

Cell culture: The human ovarian cancer OVCAR3 cell strain was cultured *in vitro* under the conditions of RPMI-1640 supplemented with 20% fetal bovine serum, 1% penicillin-streptomycin, and 10 µg/mL bovine insulin in an incubator at 37 °C with 5% CO₂. The cells were digested with a 0.25% trypsin-EDTA digestive solution once a week for passaging as per conventional practice. When the cells reached 80%-90% confluence and met the density requirement, they were harvested and counted.

Cell inoculation: 0.1 mL of the OVCAR3 cell suspension (containing 1 × 10⁷ cells in RPMI-1640:Matrigel (v/v = 1:1)) was inoculated subcutaneously into the axilla of each mouse. On day 26 after inoculation of the cells, the mice were randomly grouped and administrated based on tumor volume. The day of grouping was designated as day 0.

### Tumor measurement and experimental indices:

Tumor diameters were measured twice weekly using a vernier caliper. The tumor volume was calculated using the following formula: V = 0.5a × b², where a and b represent the long diameter and short diameter of the tumor, respectively. Mouse body weight was measured twice weekly.

The anti-tumor therapeutic effect of the test drug was evaluated by tumor growth inhibition rate TGI (%). TGI (%) = [(1 - (average tumor volume at the end of administration in a treatment group - average tumor volume at the start of administration in the treatment group)) / (average tumor volume at the end of treatment in the solvent control group - average tumor volume at the start of treatment in the solvent control group)] × 100%.

### Experimental results:

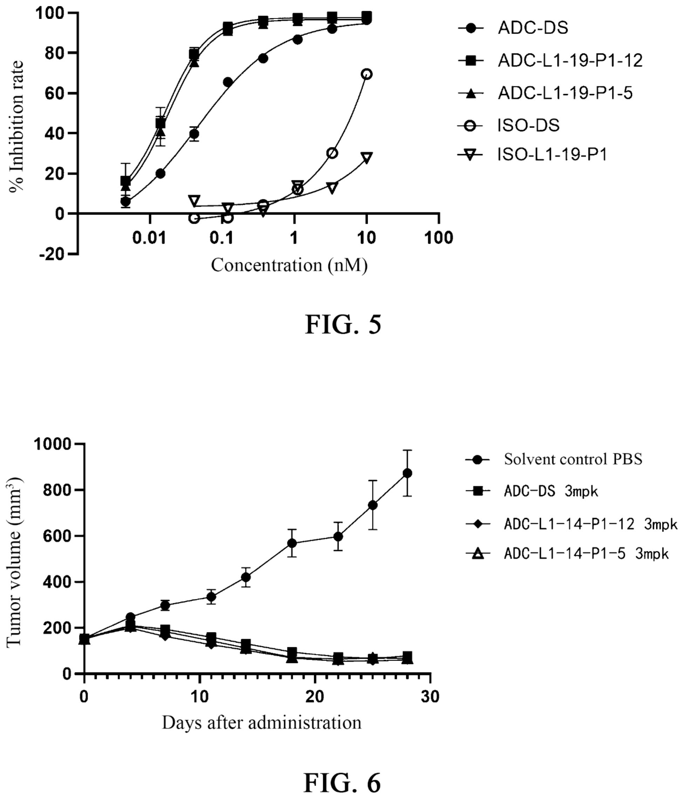

In the mouse subcutaneous xenograft tumor OVCAR3 model, both ADC-L1-14-P1-12 and ADC-L1-14-P1-5 exhibited significant inhibitory effects (P < 0.0001) on tumor growth after single intravenous administration at a dose of 3 mg/kg. The results are shown in Table 7 and FIG. 6.

**Table 7. Tumor Volume in OVCAR3 subcutaneous tumor model**

| Test drug | Dose (mg/k g) | Route of administration | Frequency of administration | Average tumor volume (mm³) | | | | | | | | | TGI (%) |
|---|---|---|---|---|---|---|---|---|---|---|---|---|---|
| | | | | Day 0 | Day4 | Day7 | Day 11 | Day 14 | Day 18 | Day22 | Day25 | Day 28 | Day 28 |
| PBS | / | | | 154. 3 | 247.1 | 297.8 | 335.8 | 420.3 | 568.8 | 598.1 | 734.3 | 873. 7 | / |
| ADC-DS | 3 | | | 154. 4 | 211.3 | 194.1 | 159.5 | 131.5 | 95.5 | 73.6 | 66.0 | 77.8 | 110. 6 |
| ADC-L1-14-P1-12 | 3 | Intravenous injection (IV) | Single | 156. 9 | 198.5 | 162.8 | 126.7 | 103.6 | 68.9 | 54.4 | 55.9 | 60.2 | 113. 4 |
| ADC-L1-14-P1-5 | 3 | | | 151. 4 | 207.0 | 183.5 | 143.4 | 113.3 | 71.3 | 64.1 | 69.7 | 66.9 | 111. 7 |

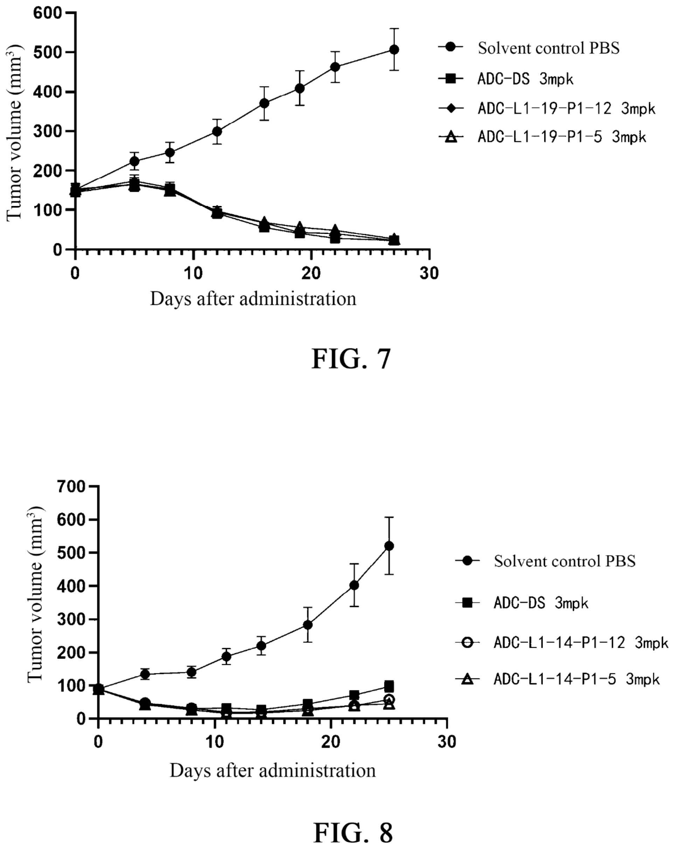

In the mouse subcutaneous xenograft tumor OVCAR3 model, both ADC-L1-19-P1-12 and ADC-L1-19-P1-5 exhibited significant inhibitory effects (P < 0.0001) on tumor growth after single intravenous administration at a dose of 3 mg/kg. The results are shown in Table 8 and FIG. 7.

**Table 8. Tumor Volume in OVCAR3 subcutaneous tumor model**

| Test drug | Dose (mg/kg) | Route of administration | Frequency of administration | Average tumor volume (mm³) | | | | | | | | TGI (%) |
|---|---|---|---|---|---|---|---|---|---|---|---|---|
| | | | | Day0 | Day5 | Day8 | Day12 | Day16 | Day 19 | Day22 | Day27 | Day27 |
| PBS (solvent control) | / | Intravenous injection (IV) | Single | 151.7 | 223.8 | 246.0 | 299.3 | 370.5 | 409.4 | 462.8 | 506.9 | / |
| ADC-DS | 3 | | | 145.0 | 165.9 | 153.4 | 91.4 | 56.2 | 41.2 | 28.9 | 23.4 | 134.2 |
| ADC-L1-19-P1-12 | 3 | | | 148.5 | 173.1 | 156.5 | 94.1 | 67.8 | 43.9 | 40.9 | 23.1 | 135.2 |
| ADC-L1-19-P1-5 | 3 | | | 153.4 | 165.0 | 149.3 | 97.3 | 68.7 | 56.6 | 49.4 | 27.3 | 135.5 |

### Example 9: Evaluation of efficacy in PA-1 subcutaneous tumor model

**Experimental reagents:** Human ovarian cancer PA-1 cells were purchased from Nanjing Cobioer Biosciences Co., Ltd. MEM medium was purchased from Gibco (Cat. No. 32561-037). Fetal bovine serum was purchased from Excell (Cat. No. FND500). Penicillin-streptomycin was purchased from Gibco (Cat. No. 15140122). NEAA was purchased from Gibco (Cat. No. 11140-050). sodium pyruvate was purchased from Gibco (Cat. No. 11360-070). Versene was purchased from Gibco (Cat. No. 15040-066). D-PBS (phosphate-buffered saline without calcium and magnesium ions) was purchased from Hyclone (Cat. No. SH30256.01). Matrigel was purchased from Corning (Cat. No. 356237).

### Experimental method:

Animal information: Balb/c nude mice (female, 5-6 weeks old, weighing about 14-20 g) were purchased from Beijing Vital River Biotechnology Co., Ltd. The mice were bred in an SPF-level environment within independently ventilated cages. All animals had *ad libitum* access to standard certified commercial laboratory diet and drinking water.

Cell culture: The human ovarian cancer PA-1 cell strain was cultured *in vitro* under the conditions of MEM supplemented with 10% fetal bovine serum, 1% penicillin-streptomycin, 1% NEAA, and 1 mM sodium pyruvate in an incubator at 37 °C with 5% CO₂. The cells were digested with a Versene digestive solution once a week for passaging as per conventional practice. When the cells reached 80%-90% confluence and met the density requirement, they were harvested and counted.

Cell inoculation: 0.1 mL of the PA-1 cell suspension (containing 1 × 10⁷ cells in MEM:Matrigel (v/v = 1:1)) was inoculated subcutaneously into the axilla of each mouse. On day 17 after inoculation of the cells, the mice were randomly grouped and administrated based on tumor volume. The day of grouping was designated as day 0.

### Tumor measurement and experimental indices:

Tumor diameters were measured twice weekly using a vernier caliper. The tumor volume was calculated using the following formula: V = 0.5a × b², where a and b represent the long diameter and short diameter of the tumor, respectively. Mouse body weight was measured twice weekly.

The anti-tumor therapeutic effect of the test drug was evaluated by tumor growth inhibition rate TGI (%). TGI (%) = [(1 - (average tumor volume at the end of administration in a treatment group - average tumor volume at the start of administration in the treatment group)) / (average tumor volume at the end of treatment in the solvent control group - average tumor volume at the start of treatment in the solvent control group)] × 100%.

### Experimental results:

In the mouse subcutaneous xenograft tumor PA-1 model, both ADC-L1-14-P1-12 and ADC-L1-14-P1-5 exhibited significant inhibitory effects (P < 0.0001) on tumor growth after single intravenous administration at a dose of 3 mg/kg. The results are shown in Table 9 and FIG. 8.

**Table 9. Tumor Volume in PA-1 subcutaneous tumor model**

| Test drug | Dose (mg/k g) | Route of administration | Frequency of administration | Average tumor volume (mm³) | | | | | | | | TGI (%) |
|---|---|---|---|---|---|---|---|---|---|---|---|---|
| | | | | Day 0 | Day4 | Day8 | Day 11 | Day 14 | Day 18 | Day22 | Day25 | Day25 |
| PBS (solvent control) | / | | | 90.3 | 134.2 | 140.7 | 187.7 | 220.5 | 283.0 | 402.8 | 520.7 | / |
| ADC-DS | 3 | Intravenous injection (IV) | Single | 88.4 | 43.0 | 29.9 | 32.7 | 26.9 | 44.7 | 71.4 | 97.4 | 97.9 |
| ADC-L1-14-P1-12 | 3 | | | 89.5 | 47.8 | 31.9 | 19.5 | 20.1 | 30.5 | 39.3 | 58.0 | 107.3 |
| ADC-L1-14-P1-5 | 3 | | | 88.8 | 42.1 | 26.4 | 15.8 | 17.4 | 24.6 | 40.0 | 45.5 | 110.1 |

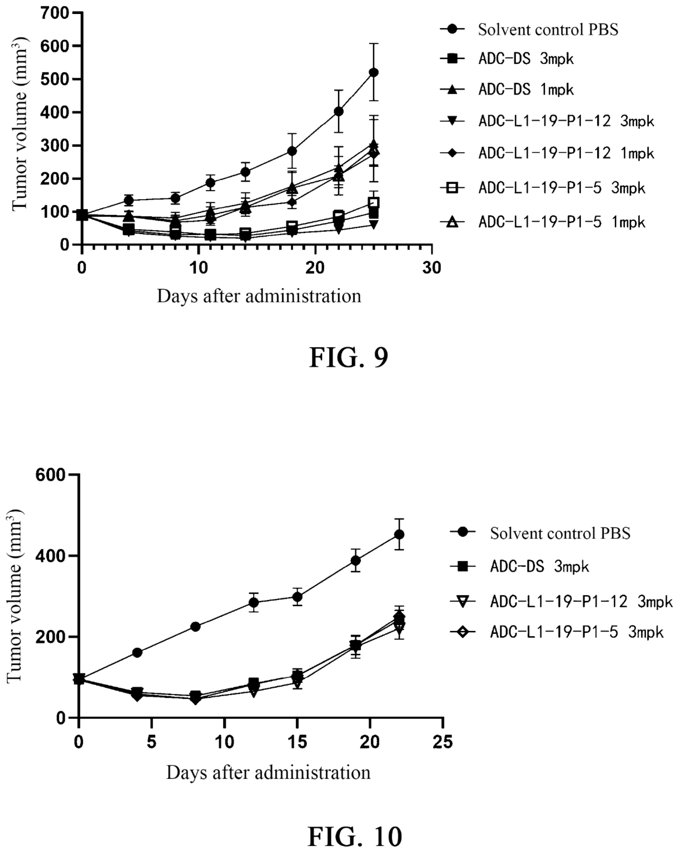

In the mouse subcutaneous xenograft tumor PA-1 model, both AUU-L1-19-P1-12 and ADC-L1-19-P1-5 exhibited significant inhibitory effects (P < 0.0001) on tumor growth after single intravenous administration at doses of 1 mg/kg and 3 mg/kg, and inhibitory effects were dose-dependent. The results are shown in Table 10 and FIG. 9.

**Table 10. Tumor Volume in PA-1 subcutaneous tumor model**

| Test drug | Dose (mg/k g) | Route of administration | Frequency of administration | Average tumor volume (mm³) | | | | | | | | TGI (%) |
|---|---|---|---|---|---|---|---|---|---|---|---|---|
| | | | | Day 0 | Day4 | Day8 | Day11 | Day 14 | Day 18 | Day22 | Day25 | Day25 |
| PBS (solvent control) | / | Intravenous injection (IV) | Single | 90.3 | 134.2 | 140. 7 | 187.7 | 220.5 | 283.0 | 402.8 | 520.7 | / |
| ADC-DS | 3 | | | 88.4 | 43.0 | 29.9 | 32.7 | 26.9 | 44.7 | 71.4 | 97.4 | 97.9 |
| | 1 | | | 90.9 | 86.0 | 80.7 | 105.6 | 125.7 | 176.6 | 234.1 | 307.8 | 49.6 |
| ADC-L1-19-P1-12 | 3 | | | 90.4 | 37.8 | 25.4 | 21.8 | 19.7 | 35.5 | 44.6 | 59.9 | 107.1 |
| | 1 | | | 86.8 | 85.5 | 68.2 | 74.2 | 113.4 | 129.2 | 209.8 | 272.4 | 56.9 |
| ADC-L1-19-P1-5 | 3 | | | 90.3 | 47.2 | 38.8 | 29.8 | 35.0 | 55.7 | 84.7 | 127.6 | 91.3 |
| | 1 | | | 90.6 | 86.9 | 72.3 | 89.6 | 113.2 | 171.4 | 208.3 | 290.3 | 53.6 |

### Example 10: Evaluation of efficacy in 786-O subcutaneous tumor model

**Experimental reagents:** Human renal cancer 786-O cells (a cell strain with low CDH6 expression) were purchased from ATCC. RPMI-1640 medium was purchased from Gibco (Cat. No. A104910). Fetal bovine serum was purchased from Excell (Cat. No. FND500). Penicillin-streptomycin was purchased from Gibco (Cat. No. 15140122). 0.25% trypsin-EDTA was purchased from Gibco (Cat. No. 25200-072). D-PBS (phosphate-buffered saline without calcium and magnesium ions) was purchased from Hyclone (Cat. No. SH30256.01). Matrigel was purchased from Corning (Cat. No. 356237).

### Experimental method:

Animal information: NOD-SCID mice (female, 8-9 weeks old, weighing about 18-25 g) were purchased from Shanghai Lingchang Biotechnology Co., Ltd. The mice were bred in an SPF-level environment within independently ventilated cages. All animals had *ad libitum* access to standard certified commercial laboratory diet and drinking water.

Cell culture: The human renal cancer 786-O cell strain was cultured *in vitro* under the conditions of RPMI-1640 supplemented with 10% fetal bovine serum and 1% penicillin-streptomycin in an incubator at 37 °C with 5% CO₂. The cells were digested with a 0.25% trypsin-EDTA digestive solution once a week for passaging as per conventional practice. When the cells reached 80%-90% confluence and met the density requirement, they were harvested and counted.

Cell inoculation: 0.1 mL of the 786-O cell suspension (containing 5 × 10⁶ cells in RPMI-1640:Matrigel (v/v = 1:1)) was inoculated subcutaneously into the axilla of each mouse. On day 13 after inoculation of the cells, the mice were randomly grouped and administrated based on tumor volume. The day of grouping was designated as day 0.

### Tumor measurement and experimental indices:

Tumor diameters were measured twice weekly using a vernier caliper. The tumor volume was calculated using the following formula: V = 0.5a × b², where a and b represent the long diameter and short diameter of the tumor, respectively. Mouse body weight was measured twice weekly.

The anti-tumor therapeutic effect of the test drug was evaluated by tumor growth inhibition rate TGI (%). TGI (%) = [(1 - (average tumor volume at the end of administration in a treatment group - average tumor volume at the start of administration in the treatment group)) / (average tumor volume at the end of treatment in the solvent control group - average tumor volume at the start of treatment in the solvent control group)] × 100%.

### Experimental results:

In the mouse subcutaneous xenograft tumor 786-O model, both ADC-L1-19-P1-12 and ADC-L1-19-P1-5 exhibited significant inhibitory effects (P < 0.0001) on tumor growth after single intravenous administration at a dose of 3 mg/kg. The results are shown in Table 11 and FIG. 10.

**Table 11. Tumor Volume in 786-O subcutaneous tumor model**

| Test drug | Dose (mg/ kg) | Route of administration | Frequency of administration | Average tumor volume (mm³) | | | | | | | TGI (%) |
|---|---|---|---|---|---|---|---|---|---|---|---|
| | | | | Day0 | Day4 | Day8 | Day12 | Day1 5 | Day1 9 | Day2 2 | Day 22 |
| PBS (solvent control) | / | | | 95.6 | 161.4 | 225.3 | 285.0 | 298.8 | 388.5 | 453.2 | / |
| ADC-DS | 3 | Intravenous injection (IV) | Single | 95.0 | 63.1 | 54.6 | 84.3 | 104.6 | 179.3 | 241.7 | 59.0 |
| ADC-L1-19-P1-12 | 3 | | | 95.9 | 59.3 | 47.1 | 65.9 | 87.0 | 175.4 | 220.9 | 65.0 |
| ADC-L1-19-P1-5 | 3 | | | 93.3 | 55.6 | 47.9 | 82.8 | 105.0 | 179.0 | 250.2 | 56.1 |

### Example 11: Assay for plasma stability of ADCs

ADC molecules (final concentration: 100 µg/mL) were separately incubated with human plasma (Oribiotech, PB021-C) and monkey plasma (Shinuoda Bio, SND-X0107) in an incubator at 37 °C. The first day of incubation was designated as day 0. Samples were then taken on days 7, 14 and 28 and assayed for free small molecules.

An internal standard working solution (300 µL, prepared in acetonitrile) was added to 20 µL of the sample, and the mixture was well mixed by vortexing for 5 min and centrifuged for 5 min (14000 rpm). Then, 4 µL of the supernatant was injected for LC-MS/MS analysis (API 6500+). The results are shown in Table 12. The results show that the ADC molecules tested were stable in both human and monkey plasma.

**Table 12. Plasma stability of CDH6-ADCs**

| Type of plasma | Incubation time (day) | Free small molecule% | | |
|---|---|---|---|---|
| | | ADC-DS | ADC-L1-19-P1-12 | ADC-L1-19-P1-5 |
| Human plasma | 0 | 0.59 | N/A | N/A |
| | 7 | 0.72 | 0.34 | 0.32 |
| | 14 | 1.21 | 0.57 | 0.54 |
| | 28 | 1.90 | 1.00 | 0.99 |
| Monkey plasma | 0 | 0.64 | N/A | N/A |
| | 7 | 2.28 | 0.35 | 0.34 |
| | 14 | 4.03 | 0.78 | 0.75 |
| | 28 | 9.66 | 1.73 | 1.53 |

| | | | | |
|---|---|---|---|---|
| N/A means that no free small molecules were detected and the free small molecule% cannot be calculated. | | | | |

## Claims

1. An antibody-drug conjugate having a structural general formula of Pc-(L-D)ₙ or a pharmaceutically acceptable salt thereof,
wherein
D is a cytotoxic drug;
L is a linker unit;
Pc is an antibody or an antigen-binding fragment thereof that specifically binds to CDH6;
the antibody or the antigen-binding fragment comprises a heavy chain variable region (VH) or/and a light chain variable region (VL), wherein the heavy chain variable region comprises HCDR1, HCDR2, and HCDR3, or/and the light chain variable region comprises LCDR1, LCDR2, and LCDR3, wherein the HCDR1-3 or/and the LCDR 1-3 are defined as follows:
(1) the HCDR1-3 are SEQ ID NOs: 11-13, or/and the LCDR1-3 are SEQ ID NOs: 14-16;
(2) the HCDR1-3 are SEQ ID NOs: 17-19, or/and the LCDR1-3 are SEQ ID NOs: 20-22;
(3) the HCDR1-3 are SEQ ID NOs: 23-25, or/and the LCDR1-3 are SEQ ID NOs: 26-28; or
the HCDR1-3 or/and the LCDR1-3 have a sequence having at least 80% identity or having at most 3 insertion, deletion, or substitution mutations compared to each CDR in the HCDR1-3 and the LCDR1-3 in any one of groups (1)-(3); optionally, the at least 80% identity is 85%, 90%, 91%, 92%, 93%, 94%, 95%, 96%, 97%, 98%, 99%, or 100% identity;
moreover, n is a real number of 1-16.

2. The antibody-drug conjugate or the pharmaceutically acceptable salt thereof according to claim 1, wherein the antibody or the antigen-binding fragment comprises a heavy chain variable region (VH) or/and a light chain variable region (VL), wherein the heavy chain variable region or/and the light chain variable region is selected from the following:
(1) the heavy chain variable region is the sequence set forth in SEQ ID NO. 1, or/and the light chain variable region is the sequence set forth in SEQ ID NO. 2;
(2) the heavy chain variable region is the sequence set forth in SEQ ID NO. 3, or/and the light chain variable region is the sequence set forth in SEQ ID NO. 4;
(3) the heavy chain variable region is the sequence set forth in SEQ ID NO. 5, or/and the light chain variable region is the sequence set forth in SEQ ID NO. 6;
or
the heavy chain variable region or/and the light chain variable region has a sequence having at least 80% identity or having at most 3 insertion, deletion, or substitution mutations compared to the heavy chain variable region or/and the light chain variable region in any one of groups (1)-(3) described above; preferably, the at least 80% identity is 85%, 90%, 91%, 92%, 93%, 94%, 95%, 96%, 97%, 98%, 99%, or 100% identity.

3. The antibody-drug conjugate or the pharmaceutically acceptable salt thereof according to any one of claims 1-2, wherein the antibody or the antigen-binding fragment comprises a heavy chain constant region sequence and/or a light chain constant region sequence; optionally, the heavy chain constant region and/or the light chain constant region is selected from an intact constant region sequence or a fragment thereof, and the constant region fragment comprises CH1, a hinge region, CH2, CH3, or Fc; optionally, the heavy chain constant region is selected from human and murine IgG1, IgG2, IgG3, and IgG4 constant regions, and the light chain constant region is selected from human and murine kappa constant regions and lambda constant regions; optionally, the antibody or the antigen-binding fragment comprises an intact heavy chain and light chain, wherein the heavy chain consists of the VH and the heavy chain constant region, the heavy chain constant region having the sequence set forth in SEQ ID NO: 9, and the light chain consists of the VL and the light chain constant region, the light chain constant region having the sequence set forth in SEQ ID NO: 10.

4. The antibody-drug conjugate or the pharmaceutically acceptable salt thereof according to any one of claims 1-3, wherein the antibody or the antigen-binding fragment is:
(1) a chimeric antibody or a fragment thereof;
(2) a humanized antibody or a fragment thereof; and/or
(3) a fully human antibody or a fragment thereof;
optionally, the antibody or the antigen-binding fragment is selected from a monoclonal antibody, a polyclonal antibody, a natural antibody, an engineered antibody, a monospecific antibody, a multispecific antibody (e.g., a bispecific antibody), a monovalent antibody, a multivalent antibody, a full-length antibody, an antibody fragment, a naked antibody, a conjugated antibody, a humanized antibody, a fully human antibody, a Fab, a Fab', an F(ab')2, an Fd, an Fv, an scFv, a diabody, and a single domain antibody.

5. The antibody-drug conjugate or the pharmaceutically acceptable salt thereof according to any one of claims 1-4, wherein the cytotoxic drug D is selected from a chemotherapeutic agent and an antibiotic; optionally, the cytotoxic drug D is selected from a DNA topoisomerase inhibitor.

6. The antibody-drug conjugate or the pharmaceutically acceptable salt thereof according to any one of claims 1-5, wherein the cytotoxic drug D is selected from a compound represented by formula (D-I): 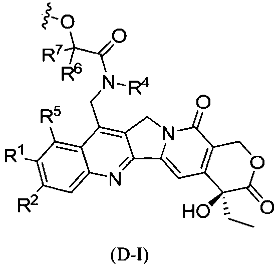 wherein
R¹ and R², together with the atoms to which they are attached, form a 5- to 6-membered heterocyclic ring, wherein the 5- to 6-membered heterocyclic ring comprises 1 or 2 oxygen atoms as ring atoms, and the 5- to 6-membered heterocyclic ring is optionally substituted with one or more D atoms;
R⁴ is selected from H and C₁-C₃ alkyl;
R⁵ is selected from H, halogen, CN, OH, NH₂, and C₁-C₃ alkyl;
R⁶ is selected from H and C₁-C₃ alkyl;
R⁷ is selected from H, C₁-C₃ alkyl, and C₃-C₆ cycloalkyl, wherein the C₁-C₃ alkyl or the C₃-C₆ cycloalkyl is optionally substituted with D, halogen, CN, =O, OH, NH₂, or C₁-C₃ alkyl.

7. The antibody-drug conjugate or the pharmaceutically acceptable salt thereof according to claim 6, wherein R¹ and R², together with the atoms to which they are attached, form 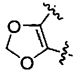 or

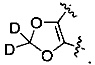

8. The antibody-drug conjugate or the pharmaceutically acceptable salt thereof according to claim 6 or 7, wherein R⁴, R⁵, and R⁶ are all selected from H.

9. The antibody-drug conjugate or the pharmaceutically acceptable salt thereof according to any one of claims 6-8, wherein R⁷ is selected from cyclopropyl.

10. The antibody-drug conjugate or the pharmaceutically acceptable salt thereof according to any one of claims 6-9, wherein the compound represented by formula (D-I) is selected from one of the following compounds: 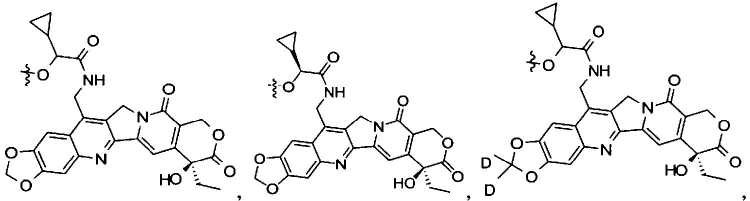 and

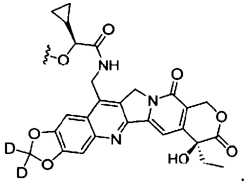

11. The antibody-drug conjugate or the pharmaceutically acceptable salt thereof according to any one of claims 1-10, wherein the linker unit L is selected from 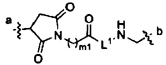 , the end a of which is covalently linked to the antibody unit Pc, and the end b of which is covalently linked to the cytotoxic drug D, wherein m1 is an integer selected from 2-8, and L¹ is selected from a peptide residue consisting of 1 to 8 amino acids, wherein the peptide residue is further optionally substituted with one or more substituents selected from halogen, CN, =O, C₁-C₆ alkyl, OH, O(C₁-C₆ alkyl), NH₂, NH(C₁-C₆ alkyl), N(C₁-C₆ alkyl)₂, C₃-C₆ cycloalkyl, and 4- to 7-membered heterocyclyl.

12. The antibody-drug conjugate or the pharmaceutically acceptable salt thereof according to claim 11, wherein L¹ is a Gly-Gly-Phe-Gly tetrapeptide residue.

13. The antibody-drug conjugate or the pharmaceutically acceptable salt thereof according to any one of claims 1-12, wherein the linker unit L is 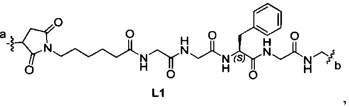 the end a of which is covalently linked to the antibody unit Pc, and the end b of which is covalently linked to the drug unit D.

14. The antibody-drug conjugate or the pharmaceutically acceptable salt thereof according to claim 1, wherein the antibody-drug conjugate or the pharmaceutically acceptable salt thereof is selected from the following antibody-drug conjugates or pharmaceutically acceptable salts thereof: 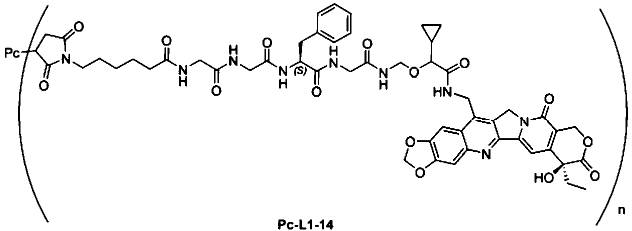 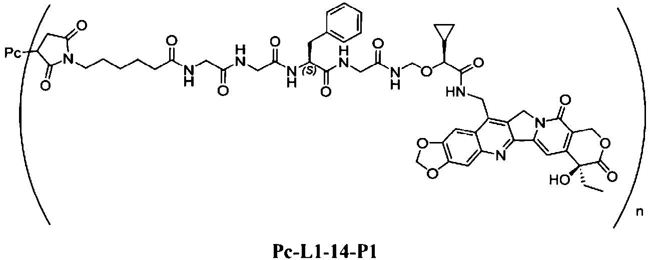 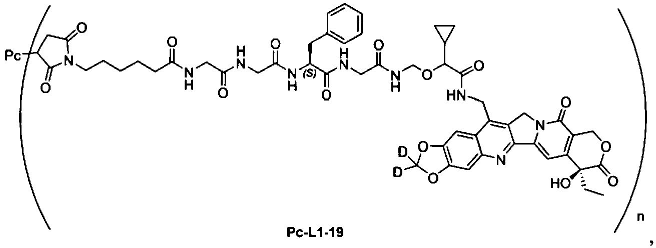 and

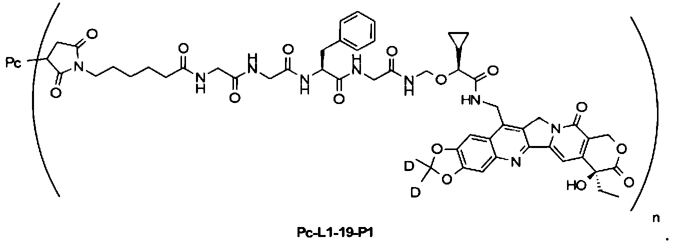

15. A pharmaceutical composition, comprising the antibody-drug conjugate or the pharmaceutically acceptable salt thereof according to any one of claims 1-14 and a pharmaceutically acceptable excipient.

16. Use of the antibody-drug conjugate or the pharmaceutically acceptable salt thereof according to any one of claims 1-14 or the pharmaceutical composition according to claim 15 in the manufacture of a medicament for treating a tumor, wherein optionally, the tumor is a CDH6-expressing tumor; optionally, the tumor is selected from ovarian cancer, renal cancer, liver cancer, soft tissue cancer, central nervous system cancer, thyroid cancer, and cholangiocarcinoma.

17. A method for treating a tumor in a mammal, comprising administering to a mammal, preferably a human, in need thereof a therapeutically effective amount of the antibody-drug conjugate or the pharmaceutically acceptable salt thereof according to any one of claims 1-14 or the pharmaceutical composition according to claim 15, wherein optionally, the tumor is a CDH6-expressing tumor; optionally, the tumor is selected from ovarian cancer, renal cancer, liver cancer, soft tissue cancer, central nervous system cancer, thyroid cancer, and cholangiocarcinoma.
